(19) Europäisches Patentamt
European Patent Office
Office européen des brevets

(11) EP 3 349 005 A1

(12) EUROPEAN PATENT APPLICATION

(43) Date of publication:
18.07.2018 Bulletin 2018/29

(51) Int Cl.:
G01N 33/487 (2006.01)

(21) Application number: 17382014.3

(22) Date of filing: 16.01.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Fundació Institut Català de
Nanociència i
Nanotecnologia
08193 Bellaterra (ES)

(72) Inventor: HOMS CORBERA, Antoni
17220 Sant Feliu de Guixols (ES)

(74) Representative: Ponti & Partners, S.L.P
C. de Consell de Cent 322
08007 Barcelona (ES)

(54) **SENSORS FOR TRANSLOCATING ANALYTES COMPRISING NANOMETER OR SUB-NANOMETER THICK HETEROSTRUCTURED FUNCTIONAL LAYERS AND A METHOD FOR SENSING TRANSLOCATING ANALYTES**

(57) The sensor of the first aspect of the invention comprises a sub-nanometer-thick or nanometer-thick functional membrane including one or more functional layers defining one or more through holes (131, 231, 331, 332, 431, 531, 631, 731, 831, 931, 1031, 1131, 1231, 1331, 1431, 1593, 1693), wherein at least one of the functional layers is a heterostructured functional layer including portions differing in that they are made of materials of different electrical properties.

Other aspects of the invention provide a slowing down of the velocity, alteration of the motion pattern, retention/stopping or re-collocation of a target translocating analyte (500, 600, 800, 1000, 1200, 1400, 1520, 1620), while passing through one or more holes (131, 231, 331, 332, 431, 531, 631, 731, 831, 931, 1031, 1131, 1231, 1331, 1431, 1593, 1693) of a functional membrane, by selectively producing dielectrophoretic forces which counter transversal forces initially governing the motion of the analyte.

Fig. 5

**Description**

FIELD OF THE INVENTION

The present invention generally relates, in a first aspect, to a sensor for translocating analytes, comprising a functional membrane with a through hole for the pass of the translocating analyte there through, and more particularly to a sensor in which the membrane comprises one or more heterostructured, preferably nanometer- or subnanometer-thick, functional layers including portions with different electrical properties.

A second aspect of the invention generally relates to a sensor for translocating analytes, comprising a functional membrane with a through hole for the pass of the translocating analyte there through, and more particularly to a sensor comprising a slow-down and manipulation mechanism to slow down the velocity of the analyte, or even stop it, when passing through the hole in a controlled manner.

For embodiments of both, the first and second aspects of the invention, the sensor comprising an analyte trapping and manipulating mechanism to control the time and space position of the analyte near or inside the through hole during its time and space controlled characterization and detection.

A third aspect of the invention generally relates to a method for sensing translocating analytes, while and result from the translocation of a target translocating analyte through a through hole defined in a functional membrane, and more particularly to a method comprising slowing down, in a controlled manner, the velocity of the target translocating analyte while passing through the hole, and/or trapping and manipulating the analyte to control the time and space position of the analyte near or inside the through hole during its time and space controlled characterization and detection.

BACKGROUND OF THE INVENTION

Nanopore, or nano-aperture/nano-orifice-based, technologies have been proposed to detect and characterize biomolecules such as nucleic acids (NA), peptides and proteins (or other macromolecules and analytes) at single molecule level. These technologies are amplification-free, neither requiring the use of fluorescent, magnetic or electronic signal amplifying labels nor the application of replication methods such as the polymerase chain reaction (PCR). Such sequencing technology is expected to greatly reduce the DNA and RNA sequencing costs and times while fostering the advent of personalized medicine. Use of similar technologies to characterize and detect other analytes of larger dimensions, such as cells, have been known and used in clinical practices since more than 50 years ago (US 2656508 A).

Electronic reading of NA sequences based on nanopore techniques include strand-sequencing by using ionic current blockade, or alternatively transverse electron current changes on the nanopore plane, resulting from the biomolecule translocation [Genomics Proteomics Bioinformatics 13, 4, (2015)]. The different structures and sizes of the five kinds of NA nucleotides (adenine (A), thymine (T), uracil (U), cytosine (C) and guanine (G)) generate different degree of pore blockades as they translocate thus resulting in different drops in the ionic current able to flow through the nanopore at a given voltage. In other words, the nanopore impedance changes, depending on the object that translocate it, and this can be used for biomolecules counting and identification using the same principle of the Counter Coulter invention (US 2656508 A).

Monitoring of these changes can potentially be used to fully sequence a NA but limitations do currently exist since nanopore-based devices suffer from signal noise, extremely short translocation times, lack of controlled translocation, mainly velocity but not limited to it, limited multiplexing, aperture dimensions and design limitations due to fabrication techniques constrains, and other issues.

Improving the fabrication procedures and reducing the environmental noise to achieve signals more repeatable and more adapted to the dimensions of the NA bases have been two of the main objectives of recent proposals in order to render these devices functional.

Membranes commonly proposed to hold the nanopore are made of materials such as $SiO_2$, $SiN_x$ or $Al_2O_3$ and have thicknesses generally greater than 10 nm. This render the nanopore NA single-base resolution sequencing quite impracticable when using methods based in monitoring blockades of the membrane traversing face-to-face ionic current. Spacing between two adjacent bases in a single-stranded DNA is about 0.3-0.7 nm and the signal produced while crossing such membranes would be dependent on the 15 bases which could be simultaneously translocate.

On the other hand, introducing electrodes integrated in the nanopore plane and measuring transverse electronic current variations in the pore have other dimensional constraints when using these materials. Consequently, in order to achieve the single-base resolution, a functional element with size or thickness comparable to the spacing between two adjacent NA nucleotides is needed to detect nucleotides one at a time. A review on these technologies can be found in a recent article [Genomics Proteomics Bioinformatics, 13, 4 (2015)].

Nanopore membranes made of mono- or multi-layer graphene (monolayer graphene has a thickness of 0.335 nm) as the functional element in the nanopore-based ionic current blockage sensors have been reported recently [Small, 5, 2638 (2009); Nano Letters, 10, 2915 (2010); Nature, 467, 190 (2010)]. However, due to the honeycomb structure of graphene, nanopore sensors made from graphene membranes suffer from large leakage currents, thus resulting in a low signal-to-noise ratio. Furthermore, due to the strong $\pi$-$\pi$ interaction, chemicals such as DNA and protein molecules in the electrolyte solution could be easily adsorbed on the graphene surface, thus affecting the sequencing detection.

Also, in US 2013/0309776 A1, a device is proposed where, for an embodiment, an electrical circuit was connected on opposite sides of the nanopore to measure flow of ionic current through the nanopore transversally in the graphene membrane plane. Limitations of this device also owns to molecules absorbance but mostly to limited sensitivity due to graphene high electrical conductance.

Furthermore, an alternative device incorporating an insulating functional sub-nanometer-thick layer without active connections was recently proposed in US2013/0037410 A1, trying to solve the graphene aperture limitations in nanopore sequencing. However, the proposed system fails to produce three-dimensional impedance readout by not solving nanopore membrane in-plane transversal measurements and being subjected to noise originated in the sample placed before and after the aperture on the sub-nanometer-thick layer.

Alternatively, nanopore devices made of stacked homogeneous layers of alternating conductive and non-conductive sub-nanometer-thick layers, forming a hetero junction, with an aperture across them, have been also proposed as NA sequencing devices [US2013/0037410 A1; US2015/0028846 A1].

On the other hand, there is also a well-known issue of uncontrolled translocation velocity in nanopore, or nano-aperture/nano-orifice -based sensors that limits the widespread application of these technologies to widespread commercial analytical devices.

It is therefore necessary to provide an alternative to the state of the art which covers the gaps found therein, and provides a solution to the above mentioned problems that the sensors of the prior art have.

DESCRIPTION OF THE INVENTION

To that end, the present relates, in a first aspect, to a sensor for translocating analytes, comprising a sub-nanometer-thick or nanometer-thick functional membrane including at least one functional layer having a thickness at a sub-nanometer scale or of up to 10 nm, said at least one functional layer defining at least one hole traversing the whole thickness of the functional membrane, wherein said at least one hole has a cross-section at a sub-nanometer or nanometer scale or with a value of up to 1 mm and is configured and arranged for allowing the pass there through of a target translocating analyte contained in a fluid coming from a first fluidic channel or first reservoir and going to a second fluidic channel or second reservoir, wherein said first and second fluidic channels or reservoirs are to be fluidically connected to the at least one hole through a cross-section larger than the cross-section of the at least one hole.

In contrast to the sensors known in the prior art, for the one proposed by the first aspect of the present invention, in a characteristic manner, the at least one functional layer is a heterostructured functional layer including portions differing to each other in that they are made of materials of different electrical properties.

In this sense, it must be highlighted that the fabrication of nanometer-thick or sub-nanometer-thick membranes made of hetero-structures was not possible until recently and there are several articles referring to how to fabricate these structures [ACS Nano, 6(10), 8583 (2012); Nano Letters, 12, 4869 (2012); Nature, 488, 627 (2012); Nature, 8, 119 (2013); Nano Letters, 13, 2668 (2013)]. Controlled fabrication of holes (such as nanopores) on these 2D hetero-structures can be performed identically to what was done in single material 2D structures, such as graphene or hexagonal Boron Nitride, previously, for example by using a TEM (Transmission Electron Microscope) or a FIB (Focused Ion Beam).

Within the context of the present application, the word "comprises" is taken to mean "includes among other things," and is not taken to mean "consists of only."

The term "hole" is taken to mean to be equivalent to "aperture", "orifice", "pore", encompassing any opening with any cross-section traversing a given material completely from side to side.

The term "analyte" is taken to mean any substance or chemical constituent that is of interest in an analytical procedure. This includes, but is not limited to, a chemical specie, a particle, a nucleic acid, a peptide, a protein, an exosome, or a cell.

The term "fluid" is taken to mean any substance or group of substances which may contain at least one analyte and that acts as carrying phase or suspending medium. This includes, but is not limited to either whole or diluted saliva, urine, or blood samples, electrolyte solutions and buffer solutions.

For some embodiments, the at least one functional layer comprises one hole while for some others it comprises several holes, arranged for example forming an array of holes.

For a preferred embodiment of the sensor of the first aspect of the invention, the at least one functional layer includes one or more electrically conductive or semiconductive portions and at least one electrically insulating or semiconducting portion.

For an implementation of said embodiment, the at least one electrically insulating or semiconducting portion is arranged between two or more of said electrically conductive or semiconductive portions.

The terms electrically "non-conductive" and "insulating" as used herein are interchangeable and have the same meaning. They are interpreted to mean "substantially electrically non-conductive."

For an embodiment, the electrically conductive portion or portions constitute respective electrodes and/or electrode connections.

According to an embodiment, the sub-nanometer-thick or nanometer-thick functional membrane includes several of the above cited functional layers in contact and stacked onto each other, wherein the hole or holes traverses the whole thickness of the stacked functional layers.

Depending on the implementation of the above embodiment, only some or all of the stacked functional layers are hetero functional layers including portions differing to each other in that they are made of materials of different electrical properties.

For a further embodiment, the sub-nanometer-thick or nanometer-thick functional membrane include at least one further functional layer in contact and stacked onto, over or under at least one of the above cited stacked functional layers, wherein said further functional layer is made of only one material having uniform electrical properties, conductive, semiconductive or insulating, and wherein the hole or holes traverse also said further functional layer.

According to an embodiment, the sensor of the first aspect of the invention comprises one or more measuring units electrically connected to the above mentioned electrodes and/or to other further electrodes connected to the above mentioned electrode connections and/or to other external further electrodes which are not in contact with the sub-nanometer-thick or nanometer-thick functional membrane, wherein the measuring unit or units are adapted to at least perform measurements of ionic current blockade and/or of transverse and/or longitudinal electron current or local impedance changes of one or more planes of the inner volume of the hole or holes, resulting from the translocation of the target translocating analyte through the hole or holes.

For an implementation of the above embodiment, the measuring unit or units comprise a control unit and real-time controlled voltage generators, for generating, under the control of the control unit time-varying voltage signals, including, but not limited to, AC voltage signals with multiple or single frequencies, DC voltage signals, or DC-AC combined voltage signals, and electric current meters to manipulate, control the motion, characterize or sequence the target translocating analyte.

For further embodiments of the sensor of the first aspect of the invention, the sensor comprises a motion generator for providing motion to the target translocating analyte to translocate through the at least one hole, wherein said motion generator comprises at least one of:

- an electrical system including at least one controlled electrical generator with outputs connected to at least two further electrodes placed respectively within said first fluidic channel or first reservoir and within said second fluidic channel or second reservoir, to provide the further electrodes with electrical signals (current or voltage) suitable to generate controlled analyte electrokinetic motion, i.e. by generating an electrophoretic force ($F_{EP}$) affecting the given analyte; and/or

- at least one controlled fluid pump (such as a mechanical, magnetical, optical or electrical pump) or analytes dragging system configured and arranged to respectively pump a solution including the target translocating analyte or to drag the target translocating analyte, sequentially through the first fluidic channel or first reservoir, through the at least one hole and through the second fluidic channel or first reservoir.

Preferably, the above mentioned electrical system also comprises at least one current meter in order to provide a more accurate control of the analyte motion based on electric current measurements performed with said at least one current meter.

For an embodiment, the above mentioned electrical system is used to apply and/or measure current or voltage signals across the pore (across planes).

Although the sensor of the first aspect of the present invention can be implemented in many different forms, for an embodiment the sensor further comprising a device comprising a housing enclosing:

- at least in part the above mentioned sub-nanometer-thick or nanometer-thick functional membrane; and

- at least the above mentioned first fluidic channel or first reservoir and the above cited second fluidic channel or second reservoir.

Additionally, for an embodiment, the sensor of the first aspect of the invention also comprises a substrate thicker than a micrometre, for structurally holding or supporting the functional layer or layers, and having at least one through orifice larger than and aligned with the at least one hole of functional layer or layers. This substrate provides the sensor with a higher mechanical resistance to be handled without breaking. Optionally, the substrate has other functions, such as providing electrical insulation, conduction or semiconducting behaviour.

Alternatively, the sensor does not include said substrate, and other elements thereof are those which provides such a mechanical resistance, such as the housing itself.

Advantageously, said device includes, within the housing, electrical connections and/or electrodes electrically connected to the above mentioned electrode(s) and/or to the electrode connection(s) constituted by the electrically conductive portion(s) of the one or more functional layers.

The sensor of the first aspect of the invention allows the selective application and/or measurement of electric voltages and currents inside the volume defined by, or/and in the vicinity of the volume defined by, the hole or holes defined in the functional layer or layers of the functional membrane.

For an embodiment of the first aspect of the present invention, the sensor comprises a slow-down and manipulation mechanism including at least one control unit and an electrical generator with outputs electrically connected to the electrode(s) and/or electrode connection(s) constituted by the electrically conductive portions of the one or more functional layers, and/or to other external further electrodes which are not in contact with the sub-nanometer-thick or nanometer-thick functional membrane, to generate and provide, under the control of the control unit, electrical signals to the electrode(s) and/or electrode connection(s) which are suitable to generate a time-controlled non-uniform electric field in the hole or holes of the one or more functional layers, and/or in the vicinity thereof, to selectively produce dielectrophoretic forces which counter transversal forces initially governing the motion of the target translocating analyte through the hole or holes, to slow down the velocity, alter the motion pattern (such as changing the direction of the analyte) retain/stop or re-collocate (in a required location within the hole or in its vicinity) the target translocating analyte (or analytes) in a controlled manner.

For an implementation of the above embodiment, the slow-down and manipulation mechanism also includes electric current meters which cooperate with the control unit to control the above mentioned generation and providing of electrical signals to control the velocity and/or position of the target translocating analyte, and thus the slowing of that velocity and/or the re-colocation of the analyte, based on electric current measurements performed with said electric current meters.

Accordingly, a method is provided to exploit the properties of the sensor of the first aspect of the invention for non-uniform electrical field generation inside each individual hole, and/or in the vicinity thereof, which allow to introduce controlled dielectrophoretic counterforces to tune translocation movements and speed. Said method including steps for performing the functions of any of the elements of the sensor described above, for any of the described embodiments.

According to an embodiment, the above mentioned real-time controlled voltage generators are adapted to generate, under the control of the one or more control units, variable controlled electric potentials and to apply the latter along time selectively to at least some of the electrodes and/or to other further electrodes connected to the electrode connections, to achieve complex variable electric fields in the hole or holes, the measuring unit or units being adapted to perform the above mentioned measurements in different three-dimensional degrees of freedom when the variable controlled electric potentials are applied between selected sensor electrodes at given times.

Hence, methods are provided to exploit the properties of the sensor of the first aspect of the invention for complex impedance 3D measurements on each individual hole which allow more accurate monitoring of hole, or holes, translocating analytes. Said methods including steps for performing the functions of any of the elements of the sensor described above, for any of the described embodiments.

For a preferred embodiment, the electrically conductive portions are made of graphene and/or Molybdenum disulphide, although other kinds of materials can also be used.

Regarding the one or more electrically insulating portions, for some embodiments they are made of boron nitride, hydrogenated graphene or graphene oxide, although other kinds of materials can also be used.

By means of the sensor of the first aspect of the invention, the multiplexing, signal-noise and in-plane transversal measurements challenges and limitations when applying the so called Counter Coulter and/or nanopore (and larger pores) technologies to the detection and characterization of analytes as well as the sequencing of biomolecules are overcome.

Regarding the hole or holes defined in the functional layer or layers of the functional membrane of the sensor of the first aspect of the invention, they can have any shape considered appropriate for their function, such as, regarding their cross-sections, a circular, elliptical or polygonal shape.

Similarly, the dimensions of each hole depends on the specific application to which the sensor is designed. Some exemplary (not limiting) dimensions are (regarding the maximum width of the cross-section of the hole, i.e. diameter if the hole has a circular cross-section): from about 1 to 5 nm when used to detect and characterize ions and small molecules, such as, but not limited to, nucleic acids, from about 5 to 50 nm when used to detect and characterize larger molecules or analytes, such as, but not limited to proteins, and nanoparticles or exosomes, from about 50 to 500 nm when used to detect and characterize entities such as virus or large exosomes, and from about 500 to 20,000 nm when used to detect and characterize entities such as bacteria or cells.

The shapes of the other holes mentioned above, i.e. those defined in the substrate, housing, fluidic channels and reservoirs, can also be any considered appropriate for their functions, while the dimension of their cross-sections in the intersection point (contact) with the functional layer or layers (i.e. at the interface with the membrane) will be always larger than the at least one hole defined in the functional layer(s).

A second aspect of the present invention relates to a sensor for translocating analytes, comprising a functional membrane including at least one functional layer defining at least one hole traversing the whole thickness of the functional membrane, wherein said at least one hole has a cross-section at a nano-meter or micro-meter scale and is configured and arranged for allowing the pass there through of a target translocating analyte coming from a first fluidic channel or first reservoir and going to a second fluidic channel or second reservoir, wherein said first and second fluidic channels or reservoirs have a cross-section larger than the cross-section of the at least one hole in the intersection point (contact) with the functional layer or layers (i.e. at the interface with the membrane).

In contrast to the sensors known in the prior art, the one proposed by the second aspect of the present invention comprises, in a characteristic manner, a slow-down and manipulation mechanism including a control unit and an electrical generator with outputs connected to electrodes configured and arranged to generate and provide, under the control of the control unit, electrical signals to the electrodes which are suitable to generate a time-controlled non-uniform electric field in the at least one hole of the at least one functional layer, and/or in the vicinity thereof, to selectively produce dielectrophoretic forces which counter transversal forces initially governing the motion of the target translocating analyte through the at least one hole, to slow down the velocity, alter the motion pattern (such as changing the direction of the analyte) retain/stop or to re-collocate (in a required location within the hole or in its vicinity) the target translocating analyte (or analytes) in a controlled manner.

The present invention also relates, in a third aspect, to a method for sensing translocating analytes, comprising:

- performing measurements of ionic current blockade and/or of transverse and/or longitudinal electron current or local impedance changes of at least one plane of the inner volume of at least one hole defined in a functional layer of a functional membrane and traversing the whole thickness of said functional membrane, wherein said at least one hole has a cross-section at a sub-nanometer or nano-meter scale or with a value of up to 1 mm,

wherein said measurements are performed while and result from the translocation or immobilization of a target translocating analyte through the at least one hole coming from a first fluidic channel or first reservoir and going to a second fluidic channel or second reservoir, wherein said first and second fluidic channels or reservoirs have a cross-section in the intersection plane with the functional layer (i.e. at the interface with the functional membrane) larger than the cross-section of the at least one hole.

In contrast to the methods known in the prior art, the one proposed by the third aspect of the present invention further comprises, in a characteristic manner, slowing down the velocity, altering the motion pattern, retaining/stopping or re-collocating the target translocating analyte, in a controlled manner, while passing through the at least one hole by generating a time-controlled non-uniform electric field in the at least one hole of the at least one functional layer, and/or in the vicinity thereof, to selectively produce dielectrophoretic forces which counter transversal forces initially governing the motion of the target translocating analyte through the at least one hole.

Regarding the second and third aspects of the present invention, and also the embodiment of the first aspect of the invention related to the slow-down and manipulation mechanism, it must be stated that in the prior art there is a well-known issue of uncontrolled translocation velocity in nanopore, or nano-aperture/nano-orifice -based sensors that limits the widespread application of these technologies to widespread commercial analytical devices.

Electrophoresis (EP) is a well-known technique to generate forces affecting the motion of charged analytes relative to a fluid under the influence of a spatially uniform electric field. The electrophoretic force ($F_{EP}$) affecting the given analyte is defined as:

$$F_{EP}=q \cdot E$$

Where q is the analyte charge and E the electric field experimented by the analyte. The direction of the force will depend on the sign of the analyte charge.

Although dielectrophoresis (DEP) is a well-known technique for manipulation, trapping and separation of analytes in non-uniform electric fields, it hasn't be used before as a slow-down and manipulation mechanism for translocating analytes. Dielectrophoretic forces ($F_{DEP}$) which move or trap analytes are generated by the effects of conduction and dielectric polarization in non-uniform electric fields. Unlike $F_{EP}$, which is determined by the net charge of the analyte, $F_{DEP}$ depends on the geometrical, conductive, and dielectric properties of the analyte, and is defined as:

$$F_{DEP}=P \cdot \nabla E \rightarrow F_{DEP}=1/2 \cdot V_{3D} \cdot Re[\alpha^*(\omega)] \cdot \nabla |E|^2 \rightarrow <F_{DEP}>=1/2 \cdot V_{3D} \cdot Re[\alpha^*(\omega)] \cdot \nabla |E_{RMS}|^2$$

Where P is the electric dipole moment of the analyte, $\nabla E$ is the electric field gradient, $\nabla|E|^2$ is the gradient of the squared modulus of the electric field, $<F_{DEP}>$ is the time-averaged $F_{DEP}$ in a given time slot, $V_{3D}$ is the analyte volume, $Re[\alpha^*(\omega)]$ is the real part of a complex number which depends on the permittivity of the analyte and the surrounding fluid which is intrinsically related to the so called Clausius-Mossotti factor (CMF) and $V|E_{RMS}|^2$ is the gradient of the squared modulus of the electric field root mean square. The direction of the force will depend on the sign of $Re[\alpha^*(\omega)]$, where $\alpha^*(\omega)$ depends on $\varepsilon_m^*$, which is the complex permittivity of the medium, $\varepsilon_p^*$, which is the complex permittivity of the analyte, and $\omega$, which is the frequency of the electric field. The complex dielectric constant or permittivity is defined as $\varepsilon^* = \varepsilon + j \cdot \sigma \cdot \omega$, where $\varepsilon$ is the dielectric constant or permittivity, $\sigma$ the conductivity, and j is the imaginary unit, sometimes also represented as "i", defined as $j^2 = i^2 = -1$.

In the ideal case of the analyte being a perfect sphere, $F_{DEP}$ can be expressed as:

$$<F_{DEP}> = 2 \cdot \pi \cdot r^3 \cdot \varepsilon_m \cdot Re[(\varepsilon_p^* - \varepsilon_a^*)/(\varepsilon_p^* + 2 \cdot \varepsilon_a^*)] \cdot \nabla|E_{RMS}|^2 = 2 \cdot \pi \cdot r^3 \cdot \varepsilon_m \cdot Re[CMF] \cdot \nabla|E_{RMS}|^2$$

Where r is the radius of the analyte, $\varepsilon_m$ is the dielectric constant or permittivity of the medium and Re[CMF] is the real part of the Clausius-Mossotti factor (CMF).

Under the sole influence of $F_{DEP}$, the analyte will be driven by $F_{DEP}$ towards an electric field extremum. The direction of the force, either towards an electrical field maximum or, alternatively, an electrical field minimum, will depend in the relation between complex conductivity of the particle and the suspending liquid. This relation will depend also of the frequency of the applied electrical field.

Due to dependence on the intrinsic properties of the analyte this phenomena can be used not only to manipulate or move analytes selectively but also to separate different analytes or an analyte from set of other compounds.

Combination of these forces, $F_{EP}$ and/or $F_{DEP}$, with other fluid, such as mechanical pumping, electrothermal flow and/or electroosmosis, and/or analytes, such as magnetic, gravitational and/or optical, dragging forces, will govern the motion of fluids and analytes according to the present invention.

BRIEF DESCRIPTION OF THE DRAWINGS

The foregoing and other features and advantages of the invention are apparent and will be more fully understood from the following detailed description of embodiments taken in conjunction with the accompanying drawings, which must be considered in an illustrative and non-limiting manner, in which:

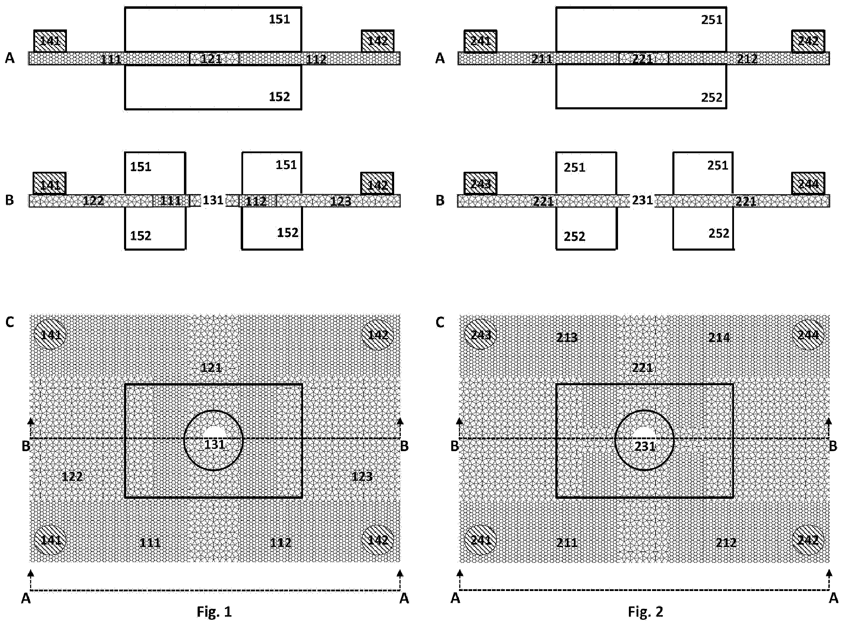

FIG. 1 illustrates cross-sectional (A and B) and top (C) views of a proposed example of a single heterostructured functional membrane, with two integrated electrodes and a single hole, embedded into a larger device aimed to manipulate, characterize or sequence molecules and analytes, for an embodiment of the sensor of the first aspect of the present invention;

FIG. 2 illustrates cross-sectional (A and B) and top (C) views of a proposed example of a single heterostructured functional membrane, with four integrated electrodes and a single hole, embedded into a larger device aimed to manipulate, characterize or sequence molecules and analytes, for an embodiment of the sensor of the first aspect of the present invention;

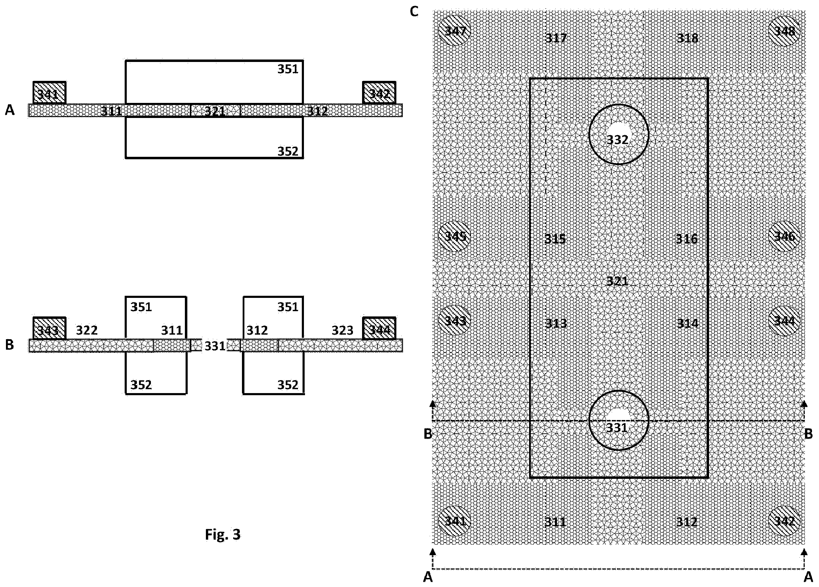

FIG. 3 illustrates cross-sectional (A and B) and top (C) views of a proposed example of a single heterostructured functional membrane, with eight integrated electrodes and two holes, embedded into a larger device aimed to manipulate, characterize or sequence molecules and analytes, for an embodiment of the sensor of the first aspect of the present invention. The membrane includes two orifices/apertures as an example of potential multiplexed sensing;

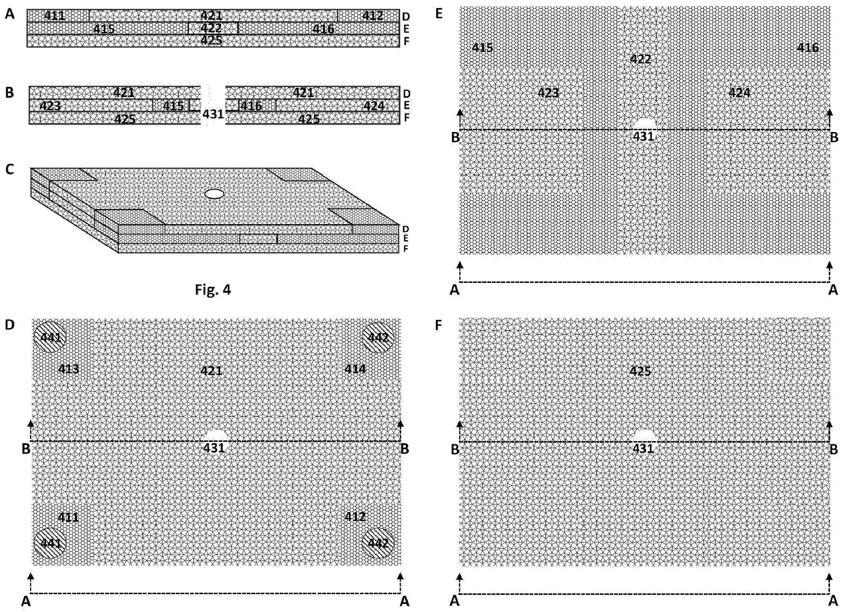

FIG. 4 illustrates cross-sectional (A and B) and 3D (C) views of a proposed example of a membrane formed by 3 stacked heterostructured functional layers (D, E, and F), with two integrated electrodes and a single hole, aimed to be embedded into a larger device to manipulate, characterize or sequence molecules and analytes, for an embodiment of the sensor of the first aspect of the present invention;

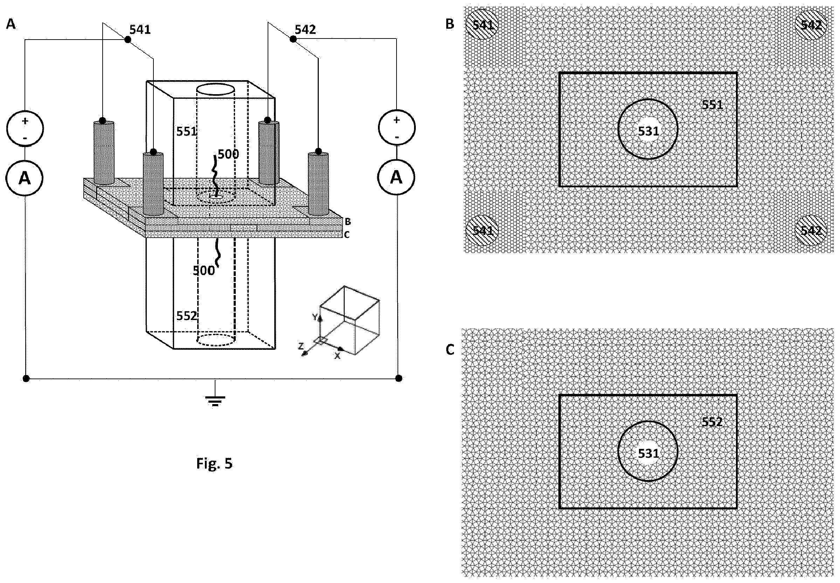

FIG. 5 illustrates 3D (A), top (B) and bottom (C) views of the example proposed in FIG. 4 of a 3 stacked heterostructured functional layers structure, with two integrated electrodes and a single hole, embedded into a larger device incorporating voltage generators and electric current meters to manipulate, characterize or sequence molecules and analytes, for an embodiment of the sensor of the first aspect of the present invention;

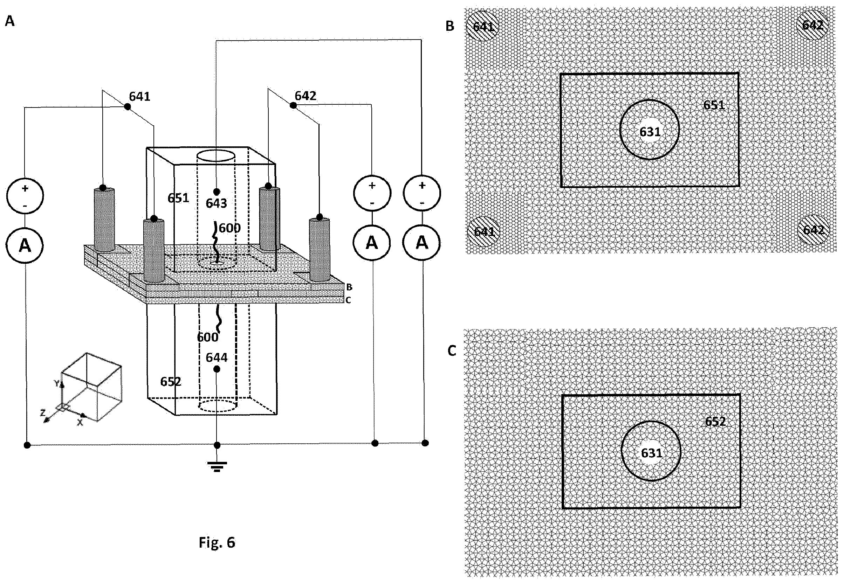

FIG. 6 illustrates 3D (A), top (B) and bottom (C) views of the example proposed in FIG. 4 of a 3 stacked heterostructured functional layers structure, with two integrated electrodes and a single hole, embedded into a larger device incorporating controlled time-dependent voltage generators and electric current meters to manipulate, characterize or sequence molecules and analytes. The device incorporates two extra external electrodes in the reservoirs to facilitate electrokinetics induced molecules and analytes translocation and current measurements, for an embodiment of the sensor of the first aspect of the present invention;

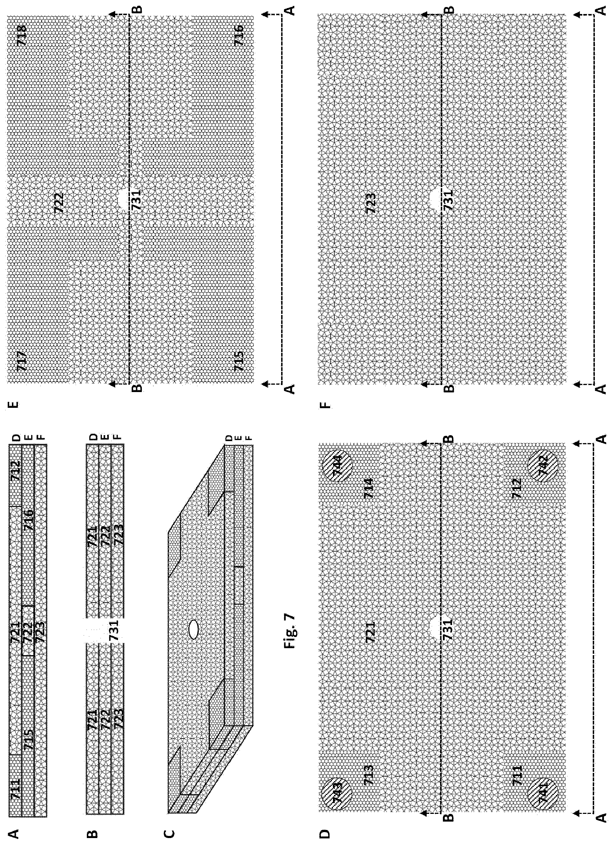

FIG. 7 illustrates cross-sectional (A and B) and 3D (C) views of a proposed example of a membrane formed by 3 stacked heterostructured functional layers (D, E, and F), with four integrated electrodes and a single hole, aimed to be embedded into a larger device to manipulate, characterize or sequence molecules and analytes, for an embodiment of the sensor of the first aspect of the present invention;

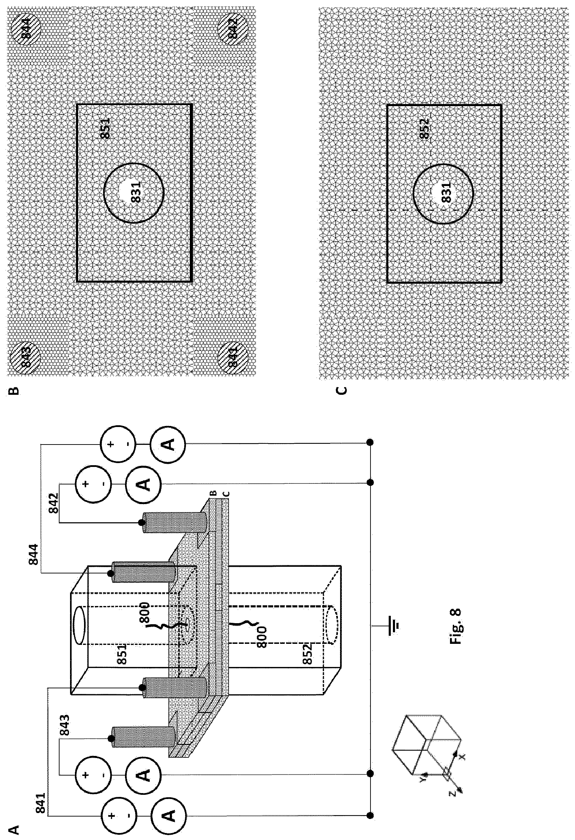

FIG. 8 illustrates 3D (A), top (B) and bottom (C) views of the example proposed in FIG. 7 of a 3 stacked heterostructured functional layers structure, with four integrated electrodes and a single hole, embedded into a larger device incorporating controlled time-dependent voltage generators and electric current meters to manipulate, characterize or sequence molecules and analytes, for an embodiment of the sensor of the first aspect of the present invention;

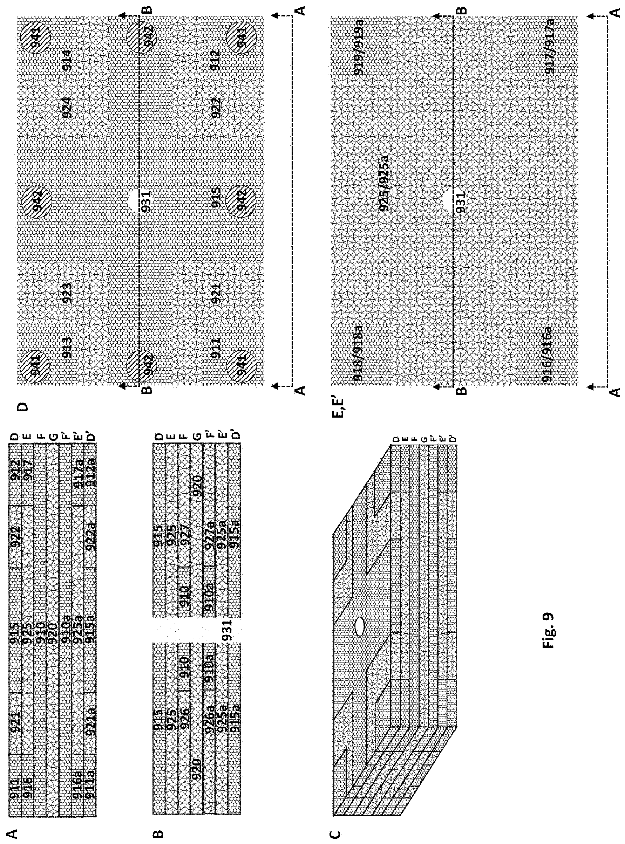

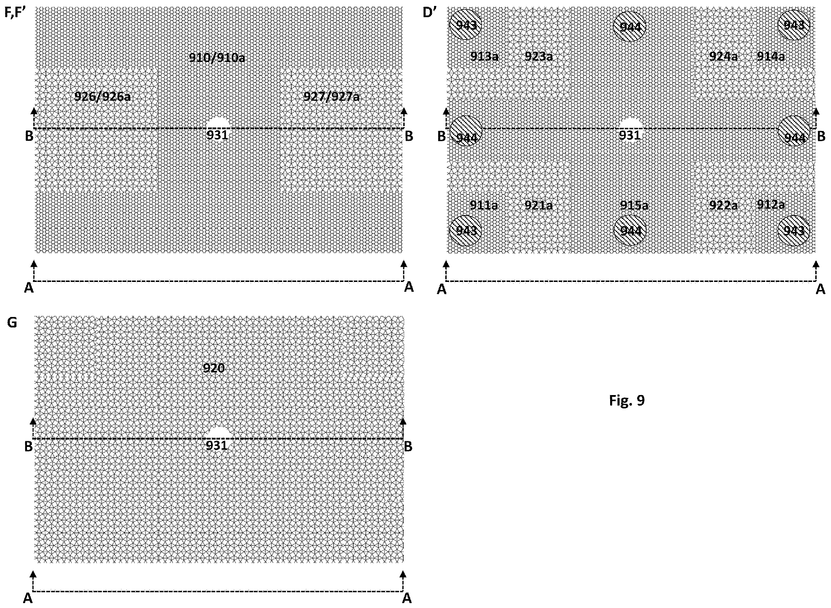

FIG. 9 illustrates cross-sectional (A and B) and 3D (C) views of a proposed example of a membrane formed by 7 stacked heterostructured functional layers (D, D', E, E', F, F' and G), with four integrated electrodes and a single hole, aimed to be embedded into a larger device to manipulate, characterize or sequence molecules and analytes, for an embodiment of the sensor of the first aspect of the present invention;

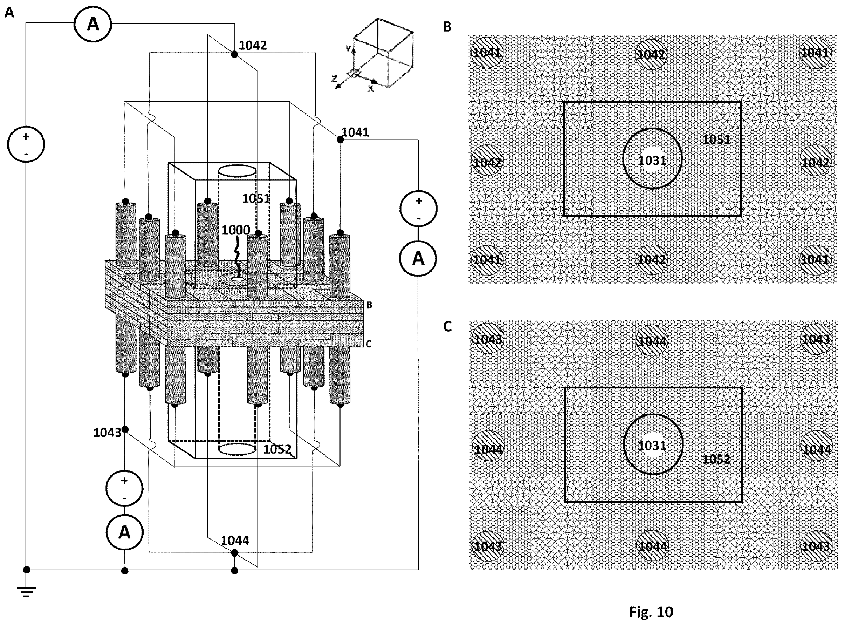

FIG. 10 illustrates 3D (A), top (B) and bottom (C) views of the example proposed in FIG. 9 of a 7 stacked heterostructured functional layers structure, with four integrated electrodes and a single hole, embedded into a larger device incorporating controlled time-dependent voltage generators and electric current meters to manipulate, characterize or sequence molecules and analytes, for an embodiment of the sensor of the first aspect of the present invention;

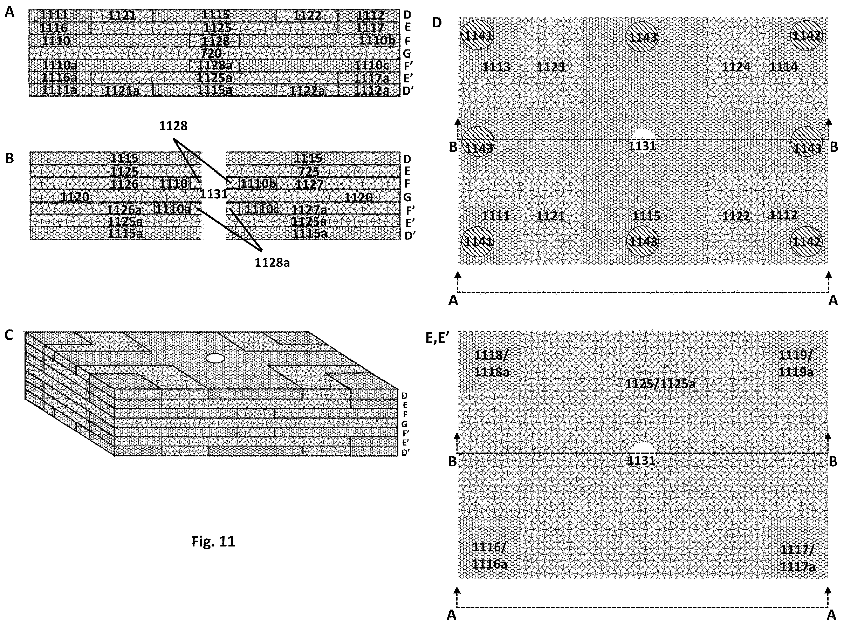

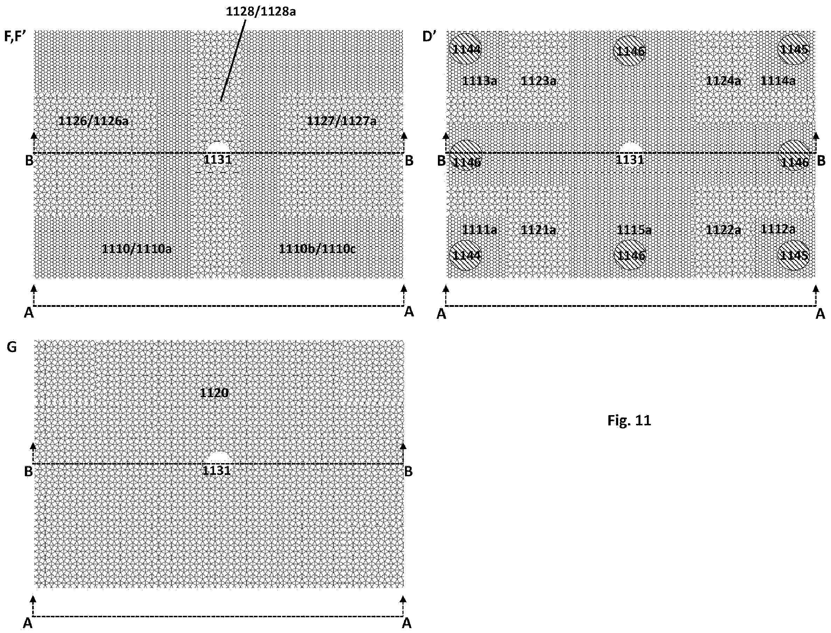

FIG. 11 illustrates cross-sectional (A and B) and 3D (C) views of a proposed example of a membrane formed by 7 stacked heterostructured functional layers (D, D', E, E', F, F' and G), with six integrated electrodes and a single hole, aimed to be embedded into a larger device to manipulate, characterize or sequence molecules and analytes, for an embodiment of the sensor of the first aspect of the present invention;

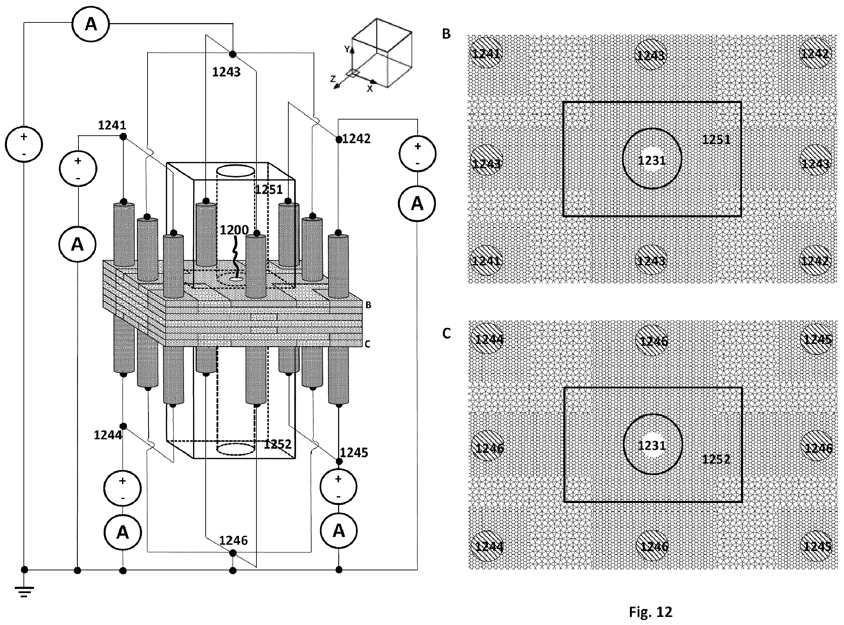

FIG. 12 illustrates 3D (A), top (B) and bottom (C) views of the example proposed in FIG. 11 of a 7 stacked heterostructured functional layers structure, with six integrated electrodes and a single hole, embedded into a larger device incorporating controlled time-dependent voltage generators and electric current meters to manipulate, characterize or sequence molecules and analytes, for an embodiment of the sensor of the first aspect of the present invention;

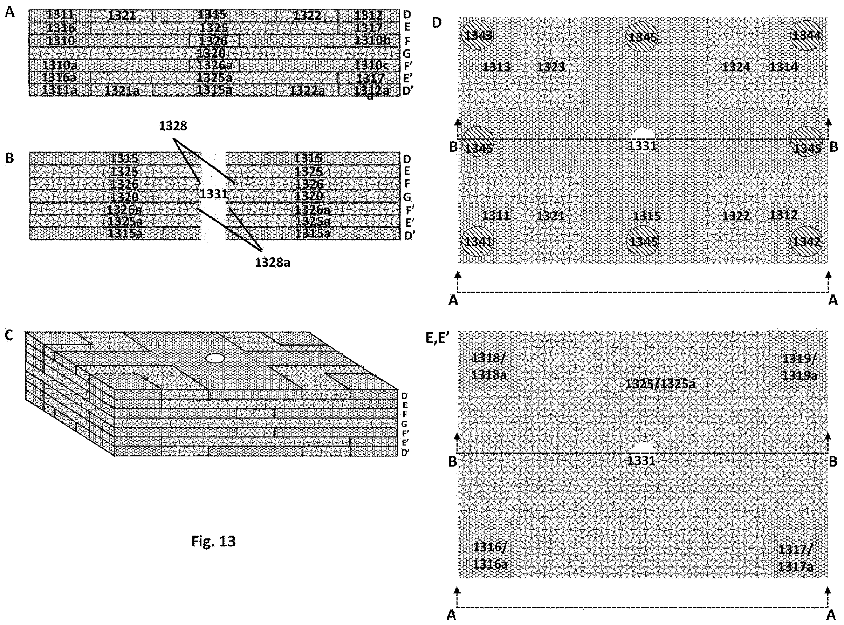

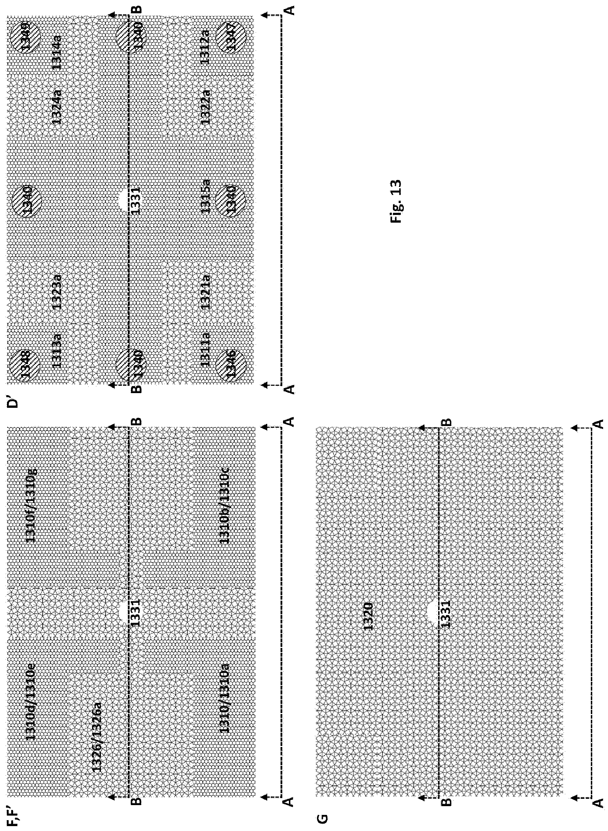

FIG. 13 illustrates cross-sectional (A and B) and 3D (C) views of a proposed example of a membrane formed by 7 stacked heterostructured functional layers (D, D', E, E', F, F' and G), with ten integrated electrodes and a single hole, aimed to be embedded into a larger device to manipulate, characterize or sequence molecules and analytes, for an embodiment of the sensor of the first aspect of the present invention;

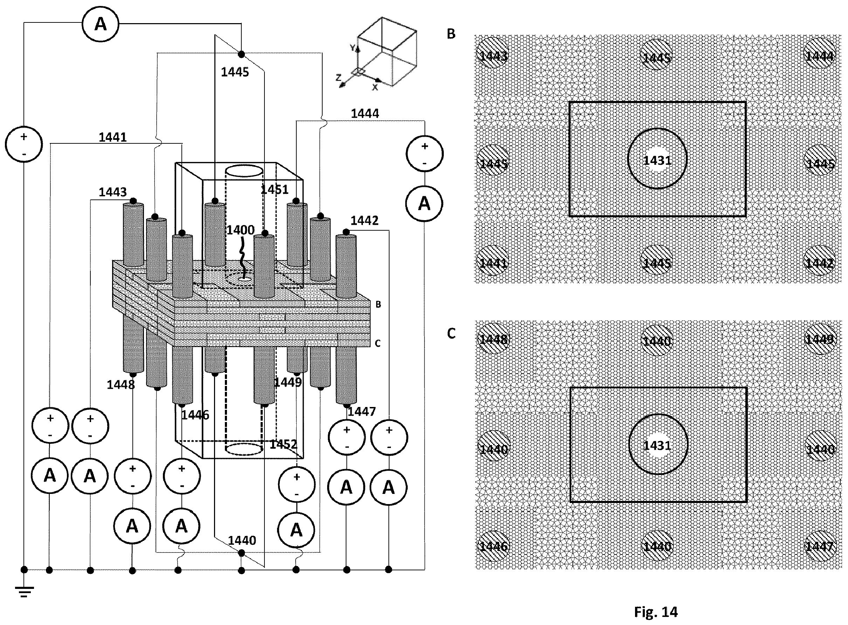

FIG. 14 illustrates 3D (A), top (B) and bottom (C) views of the example proposed in FIG. 13 of a membrane formed by 7 stacked heterostructured functional layers structure, with ten integrated electrodes and a single hole, embedded into a larger device incorporating controlled time-dependent voltage generators and electric current meters to manipulate, characterize or sequence molecules and analytes, for an embodiment of the sensor of the first aspect of the present invention;

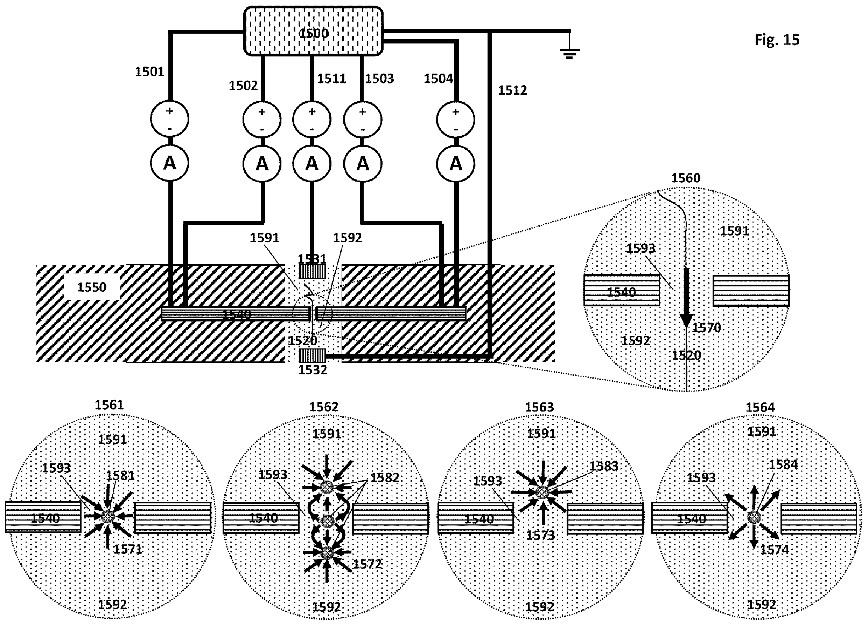

FIG. 15 illustrates a sensing device which implements an embodiment of the sensors of the first and second aspects of the present invention and which has an enclosed functional membrane, made of several stacked functional layers, which defines 4 electrodes and electrode connections. The device also incorporates two external electrodes, a control unit and 5 voltage generators and current meters. The control unit generates time-dependent controlled voltage combination patterns which are applied to the different electrodes while also performing selective impedance or electrical current measurements. External electrodes are used to generate electric fields which may contain a time-dependent continuous component (DC) in order to generate an electrophoretic driving force over charged analytes. Near the hole, manipulation of analytes position and motion through generation of dielectrophoretic traps and forces, which can be combined with simultaneous electrophoretic forces, is illustrated through electrokinetic forces and electric field extrema representations of different electrical signals and electrodes design configurations. At different times the electrical signals applied through the electrodes can be changed while also monitoring analytes translocation by means of the impedance and current measuring units thus generating time-varying forces and electrical field extrema to selectively manipulate and characterize the translocating analyte.

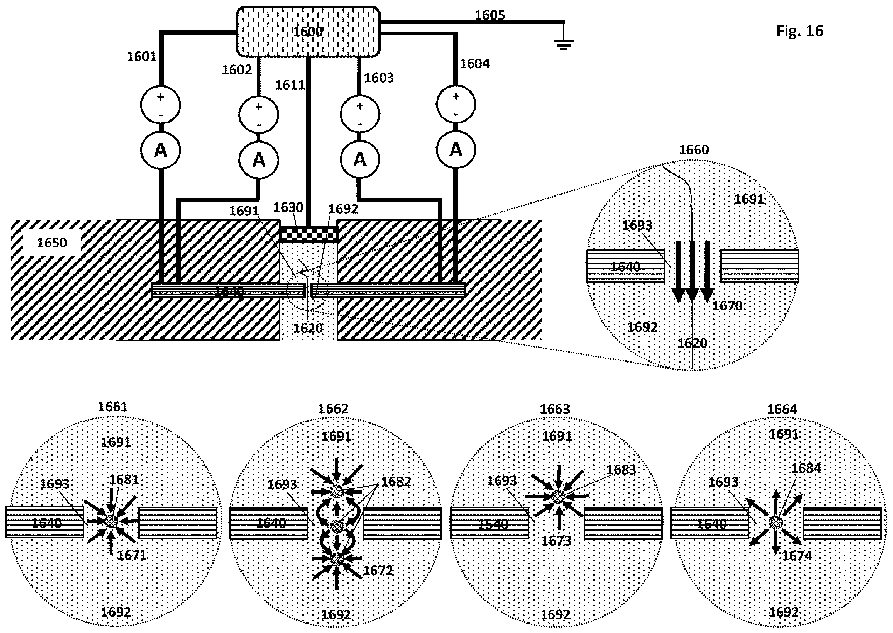

FIG. 16 illustrates a sensing device which implements a further embodiment of the sensors of the first and second aspects of the present invention and which has an enclosed functional membrane, made of several stacked functional layers, which defines 4 electrodes and electrode connections. The device also incorporates an external fluid pumping or analytes dragging system, a control unit and 4 voltage generators and current meters. The control unit generates time-dependent controlled voltage combination patterns which are applied to the different electrodes while also performing selective impedance or electrical current measurements. The hydrodynamically, magnetically or optically induced flow drags the analytes through the sensor hole while the pumping or dragging mechanism is activated by the control unit thus generating controlled analyte flows. Near the hole, manipulation of analytes position and motion through generation of dielectrophoretic traps and forces, which can be combined with simultaneous hydrodynamic, magnetic or optical dragging forces, is illustrated through electrokinetic forces and electric field extrema representations of different electrical signals and electrodes design configurations. At different times the electrical signals applied through the electrodes can be changed while also monitoring analytes translocation by means of the impedance and current measuring units thus generating time-varying forces and electrical field extrema to selectively manipulate and characterize the translocating analyte.

DETAILED DESCRIPTION OF SEVERAL EMBODIMENTS

Different embodiments of the sensor of first aspect of the present invention are shown in the appended Figures, all of them having in common that they relate to a sensor for translocating analytes, comprising a sub-nanometer-thick or nanometer-thick functional membrane including one or more functional layers, each having a thickness at a sub-nanometer scale or of up to 10 nm, and defining at least one hole 131, 231 traversing the whole thickness of the functional membrane, wherein the at least one hole 131, 231, 331, 332, 431, 531, 631, 731, 831, 931, 1031, 1131, 1231, 1331, 1431, 1593, 1693 has a cross-section at a sub-nanometer or nano-meter scale or with a value of up to 1mm and is configured and arranged for allowing the pass there through of a target translocating analyte 500, 600, 800, 1000, 1200, 1400, 1520, 1620 contained in a fluid coming from a first fluidic channel or first reservoir 151, 251, 351, 551, 651, 851, 1051, 1251, 1451, 1591, 1691 and going to a second fluidic channel or second reservoir 152, 252, 352, 552, 652, 852, 1052, 1252, 1452, 1592, 1692 wherein the first fluidic channels or first reservoir 151, 251, 351, 551, 651, 851, 1051, 1251, 1451, 1591, 1691 and the second fluidic channel or second reservoir 152, 252, 352, 552, 652, 852, 1052, 1252, 1452, 1592, 1692 are to be fluidically connected to the hole or holes through a cross-section larger than the cross-section of the hole or holes 131, 231, 331, 332, 431, 531, 631, 731, 831, 931, 1031, 1131, 1231, 1331, 1431, 1593, 1693 at the interface with the membrane.

One or more of the functional layers of the sensor of the first aspect of the invention is a heterostructured functional layer including portions differing to each other in that they are made of materials of different electrical properties.

Although in the following description of the illustrated embodiments, the membrane of the sensor of the first aspect of the invention is referred as a sub-nanometer membrane, the same drawings are valid for corresponding embodiments for which the membranes are nanometer-thick.

For the embodiment shown in FIG. 1, the sensor comprises a single heterostructured functional membrane, i.e. having only one sub-nanometer functional layer, with two integrated electrodes embedded into a larger device aimed to manipulate, characterize or sequence molecules and analytes. Figure shows: first fluidic reservoir or channel defining body 151, second fluidic reservoir or channel defining body 152, hole 131, sub-nanometer membrane conducting material (such as Graphene) part defining a first embedded electrode 111, external connection to the first electrode 141, sub-nanometer membrane insulating material (such as Boron Nitride) structure delimiting embedded electrodes 121, 122, and 123, sub-nanometer membrane conducting material part defining a second embedded electrode 112, and external connection to the second electrode 142.

The embodiment shown in FIG. 2 relates to a sensor comprising a single heterostructured functional membrane with four integrated electrodes embedded into a larger device aimed to manipulate, characterize or sequence molecules and analytes. Figure shows: first fluidic reservoir or channel defining body 251, second fluidic reservoir or channel defining body 252, hole) 231, sub-nanometer membrane conducting material part defining a first embedded electrode 211, external connection to the first electrode 241, sub-nanometer membrane insulating material structure delimiting embedded electrodes 221, sub-nanometer membrane conducting material part defining a second embedded electrode 212, external connection to the second electrode 242, sub-nanometer membrane conducting material part defining a third embedded electrode 213, external connection to the third electrode 243, sub-nanometer membrane conducting material part defining a fourth embedded electrode 214, and external connection to the fourth electrode 244;

For the embodiment shown in FIG. 3, the sensor of the first aspect of the present invention also comprises a single heterostructured functional membrane, in this case with eight integrated electrodes embedded into a larger device aimed to manipulate, characterize or sequence molecules and analytes. Figure shows: first fluidic reservoir or channel defining body 351, second fluidic reservoir or channel defining body 352, first opening hole 331, second hole 332, sub-nanometer membrane conducting material part defining a first embedded electrode 311, external connection to the first electrode 341, sub-nanometer membrane insulating material structure delimiting embedded electrodes 321, sub-nanometer membrane conducting material part defining a second embedded electrode 312, external connection to the second electrode 342, sub-nanometer membrane conducting material part defining a third embedded electrode 313, external connection to the third electrode 343, sub-nanometer membrane conducting material part defining a fourth embedded electrode 314, external connection to the fourth electrode 344, sub-nanometer membrane conducting material part defining a fifth embedded electrode 315, external connection to the fifth electrode 345; sub-nanometer membrane conducting material part defining a sixth embedded electrode 316, external connection to the sixth electrode 346; sub-nanometer membrane conducting material part defining a seventh embedded electrode 317, external connection to the seventh electrode 347; sub-nanometer membrane conducting material part defining an eighth embedded electrode 318, and external connection to the eighth electrode 348. The membrane includes two holes as an example of potential multiplexed sensing;

FIG. 4 illustrates an embodiment of the sensor of the first aspect of the present invention, for which the sensor comprises a membrane formed by 3 stacked heterostructured sub-nanometer functional layers (D, E, and F) with two integrated electrodes aimed to be embedded into a larger device to manipulate, characterize or sequence molecules and analytes. Figure shows: hole 431, sub-nanometer membrane conducting material part of the layer D defining electronic connections 411, 412, 413 and 414, first external connection 441 to electronic connections 411 and 413, second external connection 442 to electronic connections 412 and 414, sub-nanometer layer insulating material structure 421 delimiting layer D embedded electronic connections, sub-nanometer layer conducting material part of the layer E defining a first embedded electrode 415 stacked and in contact with connections 411 and 413, sub-nanometer layer conducting material part of the layer E defining a second embedded electrode 416 stacked and in contact with connections 412 and 414, sub-nanometer layer E insulating material structure 422, 423, and 424 delimiting embedded electrodes, and sub-nanometer layer F insulating material 425;

According to the embodiment shown in FIG. 5, the sensor of the first aspect of the invention comprises a membrane formed by a 3 stacked heterostructured sub-nanometer functional layers structure with two integrated electrodes embedded into a larger device incorporating real-time controlled voltage generators (which could be, but not limited to, multiple or single AC frequencies, DC, or combined generated signals) and electric current meters to manipulate, characterize or sequence molecules and analytes. Figure shows: first fluidic reservoir or channel defining body 551, second fluidic reservoir or channel defining body 552, hole 531, first external connection 541, second external connection 542, translocating analyte 500;

FIG. 6 illustrates the example proposed in FIG. 4 of a 3 stacked heterostructured sub-nanometer functional layers structure with two integrated electrodes embedded into a device incorporating real-time controlled voltage generators (which could be, but not limited to, multiple or single AC frequencies, DC, or combined generated signals) and electric current meters to manipulate, characterize or sequence molecules and analyte. The device incorporates two external electrodes in the reservoirs to facilitate electrokinetics induced molecules and analytes translocation and transversal current measurements. These also incorporate real-time controlled voltage generators (which could be, but not limited to, multiple or single AC frequencies, DC, or combined generated signals) and electric current meters which can be used in conjunction with the electrodes embedded into the device to manipulate, characterize or sequence molecules and analytes. Figure shows: first fluidic reservoir or channel defining body 651, second fluidic reservoir or channel defining body 652, hole 631, first external connection 641, second external connection 642, third external connection 643 (inside reservoir defined by 651), fourth external connection 644 (inside reservoir defined by 652), and translocating analyte 600;

FIG. 7 illustrates a proposed example of a membrane formed by a 3 stacked heterostructured sub-nanometer functional layers (D, E, and F) with four integrated electrodes aimed to be embedded into a larger device to manipulate, characterize or sequence molecules and analytes. Figure shows: hole 731, sub-nanometer layer conducting material part of the layer D defining electronic connections 711, 712, 713 and 714, first external connection 741 to electronic connection 711, second external connection 742 to electronic connection 712, third external connection 743 to electronic connection 713, fourth external connection 744 to electronic connection 714, sub-nanometer layer insulating material structure 721 delimiting layer D embedded electronic connections, sub-nanometer layer conducting material part of the layer E defining a first embedded electrode 715 stacked and in contact with connection 711, sub-nanometer layer conducting material part of the layer E defining a second embedded electrode 716 stacked and in contact with connection 712, sub-nanometer layer conducting material part of the layer E defining a third embedded electrode 717 stacked and in contact with connection 713, sub-nanometer layer conducting material part of the layer E defining a fourth embedded electrode 718 stacked and in contact with connection 714, sub-nanometer layer E insulating material structure 722 delimiting embedded electrodes, and sub-nanometer layer F insulating material 723;

FIG. 8 illustrates the example proposed in FIG. 7 of a membrane formed by a 3 stacked heterostructured sub-nanometer functional layers structure with four integrated electrodes embedded into a larger device incorporating real-time controlled voltage generators (which could be, but not limited to, multiple or single AC frequencies, DC, or combined generated signals) and electric current meters to manipulate, characterize or sequence molecules and analytes. Figure shows: first fluidic reservoir or channel defining body 851, second fluidic reservoir or channel defining body 852, hole 831, first external connection 841, second external connection 842, third external connection 843, fourth external connection 844, and translocating analyte 800;

A further embodiment of the sensor of the first aspect of the present invention is shown in FIG. 9, where the sensor comprises a membrane formed by a 3 stacked heterostructured sub-nanometer functional layers (D, D', E, E', F, F' and G) with four integrated electrodes aimed to be embedded into a larger device to manipulate, characterize or sequence molecules and analytes. Figure shows: hole 931, sub-nanometer layer conducting material part of the layer D defining electronic connections 911, 912, 913, 914 and 915, first external connection 941 to electronic connections 911, 912, 913 and 914, second external connection 942 to electronic connection and electrode 915, sub-nanometer layer insulating material structure 921 delimiting layer D embedded electronic connections 911 and 915, sub-nanometer layer insulating material structure 922 delimiting layer D embedded electronic connections 912 and 915, sub-nanometer layer insulating material structure 923 delimiting layer D embedded electronic connections 913 and 915, sub-nanometer layer insulating material structure 924 delimiting layer D embedded electronic connections 914 and 915, sub-nanometer layer conducting material part of the layer D' defining electronic connections 911 a, 912a, 913a, 914a and 915a, third external connection 943 to electronic connections 911 a, 912a, 913a and 914a, fourth external connection 944 to electronic connection and electrode 915a, sub-nanometer layer insulating material structure 921 a delimiting layer D' embedded electronic connections 911 a and 915a, sub-nanometer layer insulating material structure 922a delimiting layer D' embedded electronic connections 912a and 915a, sub-nanometer layer insulating material structure 923a delimiting layer D' embedded electronic connections 913a and 915a, sub-nanometer layer insulating material structure 924a delimiting layer D' embedded electronic connections 914a and 915a, sub-nanometer layer conducting material part of the layer E defining electronic connections 916, 917, 918, and 919, stacked and in contact with electronic connections 911, 912, 913, and 914, respectively, sub-nanometer layer insulating material structure 925 delimiting layer E embedded electronic connections, sub-nanometer layer conducting material part of the layer E' defining electronic connections 916a, 917a, 918a, and 919a, stacked and in contact with electronic connections 911 a, 912a, 913a, and 914a, respectively, sub-nanometer layer insulating material structure 925a delimiting layer E' embedded electronic connections, sub-nanometer layer conducting material part of the layer F defining electronic connection and electrode 910, stacked and in contact with electronic connections 916, 917, 918, and 919, sub-nanometer layer insulating material structures 926 and 927 delimiting layer F embedded electronic connection and electrode 910, sub-nanometer layer conducting material part of the layer F' defining electronic connection and electrode 910a, stacked and in contact with electronic connections 916a, 917a, 918a, and 919a, sub-nanometer layer insulating material structures 926a and 927a delimiting layer F' embedded electronic connection and electrode 910a, and sub-nanometer layer G insulating material 920;

FIG. 10 illustrates the example proposed in FIG. 9 of a sensor comprising a layer formed by a 7 stacked heterostructured sub-nanometer functional layers structure with four integrated electrodes embedded into a larger device incorporating real-time controlled voltage generators (which could be, but not limited to, multiple or single AC frequencies, DC, or combined generated signals) and electric current meters to manipulate, characterize or sequence molecules and analytes. Figure shows: first fluidic reservoir or channel defining body 1051, second fluidic reservoir or channel defining body 1052, hole 1031, first external connection 1041, second external connection 1042, third external connection 1043, fourth external connection 1044, and translocating analyte 1000;

A further embodiment is shown in FIG. 11, which illustrates a membrane formed by 3 stacked heterostructured sub-nanometer functional layers (D, D', E, E', F, F' and G) with six integrated electrodes aimed to be embedded into a larger device to manipulate, characterize or sequence molecules and analytes. Figure shows: hole 1131, sub-nanometer layer conducting material part of the layer D defining electronic connections 1111, 1112, 1113, 1114 and 1115, first external connection 1141 to electronic connections 1111 and 1113, second external connection 1142 to electronic connections 1112 and 1114, third external connection 1143 to electronic connection and electrode 1115, sub-nanometer layer insulating material structure 1121 delimiting layer D embedded electronic connections 1111 and 1115, sub-nanometer layer insulating material structure 1122 delimiting layer D embedded electronic connections 1112 and 1115, sub-nanometer layer insulating material structure 1123 delimiting layer D embedded electronic connections 1113 and 1115, sub-nanometer layer insulating material structure 1124 delimiting layer D embedded electronic connections 1114 and 1115, sub-nanometer layer conducting material part of the layer D' defining electronic connections 1111a, 1112a, 1113a, 1114a and 1115a, fourth external connection 1144 to electronic connections 1111 a and 1113a, fifth external connection 1145 to electronic connections 1112a and 1114a, sixth external connection 1146 to electronic connection and electrode 1115a, sub-nanometer layer insulating material structure 1121 a delimiting layer D' embedded electronic connections 1111a and 1115a, sub-nanometer layer insulating material structure 1122a delimiting layer D' embedded electronic connections 1112a and 1115a, sub-nanometer layer insulating material structure 1123a delimiting layer D' embedded electronic connections 1113a and 1115a, sub-nanometer layer insulating material structure 1124a delimiting layer D' embedded electronic connections 1114a and 1115a, sub-nanometer layer conducting material part of the layer E defining electronic connections 1116, 1117, 1118, and 1119, stacked and in contact with electronic connections 1111, 1112, 1113, and 1114, respectively, sub-nanometer layer insulating material structure 1125 delimiting layer E embedded electronic connections, sub-nanometer layer conducting material part of the layer E' defining electronic connections 1116a, 1117a, 1118a, and 1119a, stacked and in contact with electronic connections 1111a, 1112a, 1113a, and 1114a, respectively, sub-nanometer layer insulating material structure 1125a delimiting layer E' embedded electronic connections, sub-nanometer layer conducting material part of the layer F defining electronic connection and electrode 1110, stacked and in contact with electronic connections 1116 and 1118, sub-nanometer layer conducting material part of the layer F defining electronic connection and electrode 1110b, stacked and in contact with electronic connections 1117 and 1119, sub-nanometer layer insulating material structures 1126, 1127 and 1128 delimiting layer F embedded electronic connection and electrodes 1110 and 1110b, sub-nanometer layer conducting material part of the layer F' defining electronic connection and electrode 1110a, stacked and in contact with electronic connections 1116a and 1118a, sub-nanometer layer conducting material part of the layer F' defining electronic connection and electrode 1110c, stacked and in contact with electronic connections 1117a and 1119a, sub-nanometer layer insulating material structures 1126a, 1127a and 1128a delimiting layer F' embedded electronic connection and electrodes 1110a and 1110c, and sub-nanometer layer G insulating material 1120;

FIG. 12 illustrates 3D (A), top (B) and bottom (C) views of the example proposed in FIG. 11 of a 7 stacked heterostructured sub-nanometer layers structure with six integrated electrodes embedded into a larger device incorporating real-time controlled voltage generators (which could be, but not limited to, multiple or single AC frequencies, DC, or combined generated signals) and electric current meters to manipulate, characterize or sequence molecules and analytes. Figure shows: first fluidic reservoir or channel defining body 1251, second fluidic reservoir or channel defining body 1252, hole 1231, first external connection 1241, second external connection 1242, third external connection 1243, fourth external connection 1244, fifth external connection 1245, sixth external connection 1246, and translocating analyte 1200;

FIG. 13 illustrates the example of a membrane formed by 7 stacked heterostructured sub-nanometer functional layers (D, D', E, E', F, F' and G) with ten integrated electrodes aimed to be embedded into a larger device to manipulate, characterize or sequence molecules and analytes. Figure shows: hole 1331, sub-nanometer layer conducting material part of the layer D defining electronic connections 1311, 1312, 1313, 1314 and 1315, first external connection 1341 to electronic connection 1311, second external connection 1342 to electronic connection 1312, third external connection 1343 to electronic connection 1313, fourth external connection 1344 to electronic connection 1314, fifth external connection 1345 to electronic connection and electrode 1315, sub-nanometer layer insulating material structure 1321 delimiting layer D embedded electronic connections 1311 and 1315, sub-nanometer layer insulating material structure 1322 delimiting layer D embedded electronic connections 1312 and 1315, sub-nanometer layer insulating material structure 1323 delimiting layer D embedded electronic connections 1313 and 1315, sub-nanometer layer insulating material structure 1324 delimiting layer D embedded electronic connections 1314 and 1315, sub-nanometer layer conducting material part of the layer D' defining electronic connections 1311 a, 1312a, 1313a, 1314a and 1315a, sixth external connection 1346 to electronic connection 1311 a, seventh external connection 1347 to electronic connection 1312a, eighth external connection 1348 to electronic connection 1313a, ninth external connection 1349 to electronic connection 1314a, tenth external connection 1340 to electronic connection and electrode 1315a, sub-nanometer layer insulating material structure 1321 a delimiting layer D' embedded electronic connections 1311 a and 1315a, sub-nanometer layer insulating material structure 1322a delimiting layer D' embedded electronic connections 1312a and 1315a, sub-nanometer layer insulating material structure 1323a delimiting layer D' embedded electronic connections 1313a and 1315a, sub-nanometer layer insulating material structure 1324a delimiting layer D' embedded electronic connections 1314a and 1315a, sub-nanometer layer conducting material part of the layer E defining electronic connections 1316, 1317, 1318, and 1319, stacked and in contact with electronic connections 1311, 1312, 1313, and 1314, respectively, sub-nanometer layer insulating material structure 1325 delimiting layer E embedded electronic connections, sub-nanometer layer conducting material part of the layer E' defining electronic connections 1316a, 1317a, 1318a, and 1319a, stacked and in contact with electronic connections 1311a, 1312a, 1313a, and 1314a, respectively, sub-nanometer layer insulating material structure 1325a delimiting layer E' embedded electronic connections, sub-nanometer layer conducting material part of the layer F defining electronic connection and electrode 1310, stacked and in contact with electronic connection 1316, sub-nanometer layer conducting material part of the layer F defining electronic connection and electrode 1310b, stacked and in contact with electronic connection 1317, sub-nanometer layer conducting material part of the layer F defining electronic connection and electrode 1310d, stacked and in contact with electronic connection 1318, sub-nanometer layer conducting material part of the layer F defining electronic connection and electrode 1310f, stacked and in contact with electronic connection 1319, sub-nanometer layer insulating material structure 1326 delimiting layer F embedded electronic connection and electrodes 1310, 1310b, 1310d and 1310f, sub-nanometer layer conducting material part of the layer F' defining electronic connection and electrode 1310a, stacked and in contact with electronic connection 1316a, sub-nanometer layer conducting material part of the layer F' defining electronic connection and electrode 1310c, stacked and in contact with electronic connection 1317a, sub-nanometer layer conducting material part of the layer F' defining electronic connection and electrode 1310e, stacked and in contact with electronic connection 1318a, sub-nanometer layer conducting material part of the layer F' defining electronic connection and electrode 1310g, stacked and in contact with electronic connection 1319a, sub-nanometer layer insulating material structure 1326a delimiting layer F' embedded electronic connection and electrodes 1310a, 1310c, 1310e and 1310g, and sub-nanometer layer G insulating material 1320;

FIG. 14 illustrates the example proposed in FIG. 13 of a membrane formed by a 7 stacked heterostructured sub-nanometer functional layers structure with ten integrated electrodes embedded into a larger device incorporating real-time controlled voltage generators (which could be, but not limited to, multiple or single AC frequencies, DC, or combined generated signals) and electric current meters to manipulate, characterize or sequence molecules and analytes. Figure shows: first fluidic reservoir or channel defining body 1451, second fluidic reservoir or channel defining body 1452, hole 1431, first external connection 1441, second external connection 1442, third external connection 1443, fourth external connection 1444, fifth external connection 1445, sixth external connection 1446, seventh external connection 1447, eight external connection 1448, ninth external connection 1449, tenth external connection 1440, and translocating analyte 1400;

In the presented invention, analytes translocation through the sensing holes is controlled by applying different time-dependent voltages combinations at the electrodes to generate controlled uniform and/or non-uniform electric fields while performing current measurements simultaneously or sequentially. Non-uniform electric fields present local minima and maxima and generate dielectrophoretic (DEP) forces which can be used to reduce (i.e. to slow down) and control the analytes and molecules translocation velocity or the analytes standing position. DEP forces can be defined as either positive (p-DEP) or negative (n-DEP) depending on their direction, towards the electric field maximum or towards the minimum respectively (electric field traps). DEP direction depends on the frequency of the electric field and the electrical properties of the particles/molecules being manipulated and their surrounding media. Combination of different AC voltage frequencies and DC signals allow combination of electrophoresis and dielectrophoresis driven forces as well as current (or impedance) characterization of the translocating molecules and analytes. Following are several examples of devices based in the herein presented principles using dielectrophoretic forces which counter other controlled external forces:

FIG. 15 illustrates a sensing device 1550 according to the first aspect of the invention with an enclosed heterostructured functional layer 1540, made of several stacked heterostructured functional layers, which defines 4 electrodes and electrode connections 1501, 1502, 1503, and 1504. The device also incorporates two external electrodes 1531 and 1532, with its correspondent electrode connections 1511 and 1512, a control unit 1500 and 5 voltage generators and current meters. The control unit 1500 generates time-dependent controlled voltage combination patterns which are applied to the different electrodes while also performing selective impedance or electrical current measurements. External electrodes 1531 and 1532 are used to generate electric fields which may contain a time-dependent continuous component (DC) in order to generate an electrophoretic driving force 1570 arrow over charged analytes 1520. Near the hole 1560, 1561, 1562, 1563 and 1564, manipulation of analytes position and motion through controlled generation of dielectrophoretic traps 1581, 1582, 1583 and 1584, and forces 1571, 1572, 1573 and 1574 arrows (both traps and forces implementing the above mentioned slowing down of the velocity, alteration of the motion pattern, retention/stop or recollocation of the target translocating analyte), which can be combined with simultaneous electrophoretic forces 1570 arrow, is illustrated through electrokinetic forces 1570, 1571, 1572, 1573 and 1574 arrows, and electric field extrema 1581, 1582, 1583 and 1584, representations of different electrical signals and electrodes design configurations. At different times the electrical signals applied through the electrodes can be changed while also monitoring analytes translocation by means of the impedance and current measuring units thus generating time-varying forces and electrical field extrema 1581, 1582, 1583 and 1584, to selectively manipulate and characterize the translocating analyte. Figure shows: first control device 1500, first fluidic reservoir or channel defining body 1591, second fluidic reservoir or channel defining body 1592, hole 1593, first control connection to the electrodes in the functional layers 1501, second control connection to the electrodes in the functional layers 1502, third control connection to the electrodes in the functional layers 1503, fourth control connection to the electrodes in the functional layers 1504, first control connection to the external electrodes 1511, second control connection to the external electrodes 1512, functional heterostructured multilayers 1540, first external electrode 1531, second external electrode 1532, device functional heterostructured multilayers substrate or holder 1550, translocating analyte 1520, first detail of the hole and hole vicinity at a given time 1560, second detail of the hole and hole vicinity at a given time 1561, third detail of the hole and hole vicinity at a given time 1562, fourth detail of the hole and hole vicinity at a given time 1563, fifth detail of the hole and hole vicinity at a given time 1564, $F_{EP}$ direction representation when a DC electrical field is applied between 1531 and 1532, being the voltage in 1531 lower than the voltage in 1532 and 1570 a negatively charged analyte, 1570 arrow, first collection of electric field extrema positions in 1593 and its vicinity for a given combination of applied voltages 1581, second collection of electric field extrema positions in 1593 and its vicinity for a given combination of applied voltages 1582, third collection of electric field extrema positions in 1593 and its vicinity for a given combination of applied voltages 1583, fourth collection of electric field extrema positions in 1593 and its vicinity for a given combination of applied voltages 1584, first $F_{DEP}$ directions representation in 1593 and its vicinity for a given combination of applied voltages, liquid electrical properties, and analyte electrical properties 1571, second $F_{DEP}$ directions representation in 1593 and its vicinity for a given combination of applied voltages, liquid electrical properties, and analyte electrical properties 1572, third $F_{DEP}$ directions representation in 1593 and its vicinity for a given combination of applied voltages, liquid electrical properties, and analyte electrical properties 1573, and fourth $F_{DEP}$ directions representation in 1593 and its vicinity for a given combination of applied voltages, liquid electrical properties, and analyte electrical properties 1574;

A further embodiment is shown in FIG. 16, which illustrates a sensing device 1650 according to the first aspect of the invention with an enclosed heterostructured functional layer 1640, made of several stacked heterostructured functional layers, which defines 4 electrodes and electrode connections 1601, 1602, 1603, and 1604. The device also incorporates an external fluid pumping or analytes dragging system 1630 with its correspondent control line 1611, a control unit 1600 and 4 voltage generators and current meters. The control unit 1600 generates time-dependent controlled voltage combination patterns which are applied to the different electrodes while also performing selective impedance or electrical current measurements. The hydrodynamically, magnetically or optically induced flow 1670 drags the analytes through the sensor hole while the pumping or dragging mechanism 1630 is activated by the control unit 1600 thus generating controlled analyte flow. Near the hole 1660, 1661, 1662, 1663 and 1664, manipulation of analytes position and motion through controlled generation of dielectrophoretic traps 1681, 1682, 1683 and 1684, and forces 1671, 1672, 1673 and 1674 arrows (both raps and forces implementing the above mentioned slowing down of the velocity, alteration of the motion pattern, retention/stop or re-collocation of the target translocating analyte), which can be combined with simultaneous external forces 1670 arrow, is illustrated through electrokinetic forces 1670, 1671, 1672, 1673 and 1674 arrows, and electric field extrema 1681, 1682, 1683 and 1684, representations of different electrical signals and electrodes design configurations. At different times the electrical signals applied through the electrodes can be changed while also monitoring analytes translocation by means of the impedance and current measuring units thus generating time-varying forces and electrical field extrema 1681, 1682, 1683 and 1684, to selectively manipulate and characterize the translocating analyte 1620. Figure shows: first control device 1600, first fluidic reservoir or channel defining body 1691, second fluidic reservoir or channel defining body 1692, hole 1693, first control connection to the electrodes in the functional layers 1601, second control connection to the electrodes in the functional layers 1602, third control connection to the electrodes in the functional layers 1603, fourth control connection to the electrodes in the functional layers 1604, first control connection to a voltage reference 1605, first control connection to the external pumping mechanism 1611, functional heterostructured multilayers 1640, first external pumping mechanism 1630, device functional heterostructured multilayers substrate or holder 1650, translocating analyte 1620, first detail of the hole and hole vicinity at a given time 1660, second detail of the hole and hole vicinity at a given time 1661, third detail of the hole and hole vicinity at a given time 1662, fourth detail of the hole and hole vicinity at a given time 1663, fifth detail of the hole and hole vicinity at a given time 1664, mechanical, magnetical or optical dragging force direction representation when the force is applied through 1630 actuation, 1670 arrow, first collection of electric field extrema positions in 1693 and its vicinity for a given combination of applied voltages 1681, second collection of electric field extrema positions in 1693 and its vicinity for a given combination of applied voltages 1682, third collection of electric field extrema positions in 1693 and its vicinity for a given combination of applied voltages 1683, fourth collection of electric field extrema positions in 1693 and its vicinity for a given combination of applied voltages 1684, first $F_{DEP}$ directions representation in 1693 and its vicinity for a given combination of applied voltages, liquid electrical properties, and analyte electrical properties 1671, second $F_{DEP}$ directions representation in 1693 and its vicinity for a given combination of applied voltages, liquid electrical properties, and analyte electrical properties 1672, third $F_{DEP}$ directions representation in 1693 and its vicinity for a given combination of applied voltages, liquid electrical properties, and analyte electrical properties 1673, and fourth $F_{DEP}$ directions representation in 1693 and its vicinity for a given combination of applied voltages, liquid electrical properties, and analyte electrical properties 1674.

A person skilled in the art could introduce changes and modifications in the embodiments described without departing from the scope of the invention as it is defined in the attached claims.

For example, those skilled in the art will further appreciate that the present invention may use different shapes, not only rectangular, for the nanonometer-thick and sub-nanometer thick membranes embedded electrodes.

Accordingly, the present invention is not limited to any particular geometrical shape of the embedded electrodes. Rather, reference should be made to the appended claims as indicative of the scope and content of the present invention.

As a further example, those skilled in the art will also further appreciate that the present invention may use other controlled time-dependent voltage combinations and distributions without departing from the spirit or central attributes thereof. In that the foregoing description of the present invention discloses only exemplary cases thereof, it is to be understood that other variations are contemplated as being within the scope of the present invention.

Accordingly, the present invention is not limited in the particular relative electric voltages combinations which have been described in detail therein. Rather, reference should be made to the appended claims as indicative of the scope and content of the present invention.

**Claims**

1.  A sensor for translocating analytes, comprising a sub-nanometer-thick or nanometer-thick functional membrane including at least one functional layer having a thickness at a sub-nanometer scale or of up to 10 nm, said at least one functional layer defining at least one hole (131, 231, 331, 332, 431, 531, 631, 731, 831, 931, 1031, 1131, 1231, 1331, 1431, 1593, 1693) traversing the whole thickness of the functional membrane, wherein said at least one hole (131, 231, 331, 332, 431, 531, 631, 731, 831, 931, 1031, 1131, 1231, 1331, 1431, 1593, 1693) has a cross-section at a sub-nanometer or nano-meter scale or with a value of up to 1mm and is configured and arranged for allowing the pass there through of a target translocating analyte (500, 600, 800, 1000, 1200, 1400, 1520, 1620) contained in a fluid coming from a first fluidic channel or first reservoir (151, 251, 351, 551, 651, 851, 1051, 1251, 1451, 1591, 1691) and going to a second fluidic channel or second reservoir (152, 252, 352, 552, 652, 852, 1052, 1252, 1452, 1592, 1692), wherein said first (151, 251, 351, 551, 651, 851, 1051, 1251, 1451, 1591, 1691) and second (152, 252, 352, 552, 652, 852, 1052, 1252, 1452, 1592, 1692) fluidic channels or reservoirs are to be fluidically connected to the at least one hole through a cross-section larger than the cross-section of the at least one hole (131, 231, 331, 332, 431, 531, 631, 731, 831, 931, 1031, 1131, 1231, 1331, 1431, 1593, 1693) at the interface with the functional membrane, **characterised in that** said at least one functional layer is a heterostructured functional layer including portions differing to each other **in that** they are made of materials of different electrical properties.

2. A sensor according to claim 1, wherein the at least one functional layer includes at least one electrically conductive or semiconductive portion (111, 112, 211, 212, 213, 214, 311, 312, 313, 314, 315, 316, 317, 318, 411, 412, 413, 414, 415, 416, 711, 712, 713, 714, 715, 716, 717, 718, 910, 910a, 911, 912, 913, 914, 915, 911 a, 912a, 913a, 914a, 915a, 916, 917, 918, 919, 916a, 917a, 918a, 919a, 1110, 1110a, 1110b, 1110c, 1111, 1112, 1113, 1114, 1115, 1111a, 1112a, 1113a, 1114a, 1115a, 1116, 1117, 1118, 1119, 1116a, 1117a 1118a, 1119a, 1310, 1310a, 1310b, 1310c, 1310d, 1310e, 1310f, 1310g, 1311, 1312, 1313, 1314, 1315, 1311a, 1312a, 1313a, 1314a, 1315a, 1316, 1317, 1318, 1319, 1316a, 1317a 1318a, 1319a) and at least one electrically isolating or semiconducting portion (121, 122, 123, 221, 321, 421, 422, 423, 424, 425, 551, 552, 651, 652, 721, 722, 723, 851, 852, 920, 921, 922, 923, 924, 925, 926, 927, 921 a, 922a, 923a, 924a, 925a, 925a, 926a, 927a, 1120, 1121, 1122, 1123, 1124, 1125, 1126, 1127, 1121a, 1122a, 1123a, 1124a, 1125a, 1126a, 1127a, 1320, 1321, 1322, 1323, 1324, 1325, 1326, 1321a, 1322a, 1323a, 1324a, 1325a, 1326a).

3. A sensor according to claim 2, wherein said at least one electrically conductive portion (111, 112, 211, 212, 213, 214, 311, 312, 313, 314, 315, 316, 317, 318, 411, 412, 413, 414, 415, 416, 711, 712, 713, 714, 715, 716, 717, 718, 910, 910a, 911, 912, 913, 914, 915, 911a, 912a, 913a, 914a, 915a, 916, 917, 918, 919, 916a, 917a, 918a, 919a, 1110, 1110a, 1110b, 1110c, 1111, 1112, 1113, 1114, 1115, 1111a, 1112a, 1113a, 1114a, 1115a, 1116, 1117, 1118, 1119, 1116a, 1117a 1118a, 1119a, 1310, 1310a, 1310b, 1310c, 1310d, 1310e, 1310f, 1310g, 1311, 1312, 1313, 1314, 1315, 1311a, 1312a, 1313a, 1314a, 1315a, 1316, 1317, 1318, 1319, 1316a, 1317a 1318a, 1319a) constitute at least one electrode and/or electrode connection.

4. A sensor according to any of the previous claims, wherein the sub-nanometer-thick or nanometer-thick functional membrane includes several of said functional layers in contact and stacked onto each other, wherein said at least on hole (131, 231, 331, 332, 431, 531, 631, 731, 831, 931, 1031, 1131, 1231, 1331, 1431, 1593, 1693) traverses the whole thickness of the stacked functional layers.

5. A sensor according to claim 4, wherein the sub-nanometer-thick or nanometer-thick functional membrane include at least one further functional layer in contact and stacked onto, over or under one of said stacked functional layers, wherein said further functional layer is made of only one material having uniform electrical properties, conductive, semiconductive or insulating, and wherein the at least one hole (131, 231, 331, 332, 431, 531, 631, 731, 831, 931, 1031, 1131, 1231, 1331, 1431, 1593, 1693) traverses also said further functional layer.

6. A sensor according to claim 3 or any of claims 4 to 5 when depending on claim 3, comprising at least one measuring unit electrically connected to said at least one electrode and/or to other further electrodes (141, 142, 241, 242, 243, 244, 341, 342, 343, 344, 345, 346, 347, 348, 441, 442, 541, 542, 641, 642, 741, 742, 743, 744, 841, 842, 843, 844, 941, 942, 943, 944, 1041, 1042, 1043, 1044, 1141, 1142, 1143, 1144, 1145, 1146, 1241, 1242, 1243, 1244, 1245, 1246, 1340, 1341, 1342, 1343, 1344, 1345, 1346, 1347, 1348, 1349, 1440, 1441, 1442, 1443, 1444, 1445, 1446, 1447, 1448, 1449) connected to said electrode connections and/or to other external further electrodes (643, 644) which are not in contact with the sub-nanometer-thick or nanometer-thick functional membrane, wherein said at least one measuring unit is adapted to at least perform measurements of ionic current blockade and/or of transverse and/or longitudinal electron current or local impedance changes of at least one plane of the inner volume of the at least one hole, resulting from the translocation of the target translocating analyte (500, 600, 800, 1000, 1200, 1400, 1520, 1620) through the at least one hole (131, 231, 331, 332, 431, 531, 631, 731, 831, 931, 1031, 1131, 1231, 1331, 1431, 1593, 1693).

7. A sensor according to claim 6, wherein said at least one measuring unit comprises a control unit and real-time controlled voltage generators, for generating, under the control of the control unit time-varying voltage signals, including AC voltage signals with multiple or single frequencies, DC voltage signals, or DC-AC combined voltage signals, and electric current meters (A) to manipulate, control the motion, characterize or sequence the target translocating analyte (500, 600, 800, 1000, 1200, 1400, 1520, 1620).

8. A sensor according to claim 3 or any of claims 4 to 7 when depending on claim 3, comprising a motion generator for providing motion to the target translocating analyte (500, 600, 800, 1000, 1200, 1400, 1520, 1620) to translocate through the at least one hole (131, 231, 331, 332, 431, 531, 631, 731, 831, 931, 1031, 1131, 1231, 1331, 1431, 1593, 1693), wherein said motion generator comprises at least one of:

- an electrical system including at least one controlled electrical generator with outputs connected to at least two further electrodes (643, 644, 1531, 1532) placed respectively within said first fluidic channel or first reservoir (151, 251, 351, 551, 651, 851, 1051, 1251, 1451, 1591, 1691) and within said second fluidic channel or second reservoir (152, 252, 352, 552, 652, 852, 1052, 1252, 1452, 1592, 1692), to provide the further electrodes (643, 644, 1531, 1532) with electrical signals suitable to generate controlled analyte electrokinetic motion; and/or
- at least one controlled fluid pump or analytes dragging system (1630) configured and arranged respectively to pump a solution including the target translocating analyte (500, 600, 800, 1000, 1200, 1400, 1620) or to drag the target translocating analyte (500, 600, 800, 1000, 1200, 1400, 1620) itself, sequentially through the first fluidic channel or first reservoir (151, 251, 351, 551, 651, 851, 1051, 1251, 1451, 1591, 1691), through the at least one hole (131, 231, 331, 332, 431, 531, 631, 731, 831, 931, 1031, 1131, 1231, 1331, 1431, 1593, 1693) and through the second fluidic channel or second reservoir (152, 252, 352, 552, 652, 852, 1052, 1252, 1452, 1592, 1692).

9. A sensor according to any of the previous claims, further comprising a device comprising a housing enclosing:

- at least in part said sub-nanometer-thick or nanometer-thick functional membrane; and
- at least said first fluidic channel or first reservoir (151, 251, 351, 551, 651, 851, 1051, 1251, 1451, 1591, 1691) and said second fluidic channel or second reservoir (152, 252, 352, 552, 652, 852, 1052, 1252, 1452, 1592, 1692).

10. A sensor according to claim 9 when depending on claim 3, wherein said device includes, within said housing, electrical connections and/or electrodes electrically connected to said at least one electrode and/or electrode connection constituted by the electrically conductive portions (111, 112, 211, 212, 213, 214, 311, 312, 313, 314, 315, 316, 317, 318, 411, 412, 413, 414, 415, 416, 711, 712, 713, 714, 715, 716, 717, 718, 910, 910a, 911, 912, 913, 914, 915, 911a, 912a, 913a, 914a, 915a, 916, 917, 918, 919, 916a, 917a, 918a, 919a, 1110, 1110a, 1110b, 1110c, 1111, 1112, 1113, 1114, 1115, 1111a, 1112a, 1113a, 1114a, 1115a, 1116, 1117, 1118, 1119, 1116a, 1117a 1118a, 1119a, 1310, 1310a, 1310b, 1310c, 1310d, 1310e, 1310f, 1310g, 1311, 1312, 1313, 1314, 1315, 1311a, 1312a, 1313a, 1314a, 1315a, 1316, 1317, 1318, 1319, 1316a, 1317a 1318a, 1319a) of the at least one functional layer.

11. A sensor according to claim 3 or to any of claims 4 to 10 when depending on claim 3, comprising a slow-down and manipulation mechanism including at least one control unit (1500, 1600) and an electrical generator with outputs electrically connected to the at least one electrode and/or electrode connection constituted by the electrically conductive portions (111, 112, 211, 212, 213, 214, 311, 312, 313, 314, 315, 316, 317, 318, 411, 412, 413, 414, 415, 416, 711, 712, 713, 714, 715, 716, 717, 718, 910, 910a, 911, 912, 913, 914, 915, 911a, 912a, 913a, 914a, 915a, 916, 917, 918, 919, 916a, 917a, 918a, 919a, 1110, 1110a, 1110b, 1110c, 1111, 1112, 1113, 1114, 1115, 1111a, 1112a, 1113a, 1114a, 1115a, 1116, 1117, 1118, 1119, 1116a, 1117a 1118a, 1119a, 1310, 1310a, 1310b, 1310c, 1310d, 1310e, 1310f, 1310g, 1311, 1312, 1313, 1314, 1315, 1311a, 1312a, 1313a, 1314a, 1315a, 1316, 1317, 1318, 1319, 1316a, 1317a 1318a, 1319a) of the at least one functional layer, and/or to other external further electrodes (643, 644, 1531, 1532) which are not in contact with the sub-nanometer-thick or nanometer-thick functional membrane, to generate and provide, under the control of the control unit, electrical signals to the at least one electrode and/or electrode connection which are suitable to generate a time-controlled non-uniform electric field in the at least hole (131, 231, 331, 332, 431, 531, 631, 731, 831, 931, 1031, 1131, 1231, 1331, 1431, 1593, 1693) of the at least one functional layer, and/or in the vicinity thereof, to selectively produce dielectrophoretic forces which counter transversal forces initially governing the motion of the target translocating analyte (500, 600, 800, 1000, 1200, 1400, 1520, 1620) through the at least one hole (131, 231, 331, 332, 431, 531, 631, 731, 831, 931, 1031, 1131, 1231, 1331, 1431, 1593, 1693), to slow down the velocity, alter the motion pattern, retain/stop or re-collocate the target translocating analyte (500, 600, 800, 1000, 1200, 1400, 1520, 1620) in a controlled manner.

12. A sensor according to claim 7 or to any of claims 8 to 11 when depending on claim 7, wherein said real-time controlled voltage generators are adapted to generate, under the control of the at least one control unit, variable controlled electric potentials and to apply the latter along time selectively to at least some of the at least one electrode and/or to other further electrodes (141, 142, 241, 242, 243, 244, 341, 342, 343, 344, 345, 346, 347, 348, 441, 442, 541, 542, 641, 642, 643, 644 741, 742, 743, 744, 841, 842, 843, 844, 941, 942, 943, 944, 1041, 1042, 1043, 1044, 1141, 1142, 1143, 1144, 1145, 1146, 1241, 1242, 1243, 1244, 1245, 1246, 1340, 1341, 1342, 1343, 1344, 1345, 1346, 1347, 1348, 1349, 1440, 1441, 1442, 1443, 1444, 1445, 1446, 1447, 1448, 1449) connected to the electrode connections, to achieve complex variable electric fields in the at least one hole (131, 231, 331, 332, 431, 531, 631, 731, 831, 931, 1031, 1131, 1231, 1331, 1431), the measuring unit being adapted to perform said measurements in different three-dimensional degrees of freedom when said variable controlled electric potentials are applied between selected sensor electrodes at given times.

13. A sensor according to claim 2 or to any of claims 3 to 12 when depending on claim 2, wherein electrically conductive portions (111, 112, 211, 212, 213, 214, 311, 312, 313, 314, 315, 316, 317, 318, 411, 412, 413, 414, 415, 416, 711, 712, 713, 714, 715, 716, 717, 718, 910, 910a, 911, 912, 913, 914, 915, 911 a, 912a, 913a, 914a, 915a, 916, 917, 918, 919, 916a, 917a, 918a, 919a, 1110, 1110a, 1110b, 1110c, 1111, 1112, 1113, 1114, 1115, 1111a, 1112a, 1113a, 1114a, 1115a, 1116, 1117, 1118, 1119, 1116a, 1117a 1118a, 1119a, 1310, 1310a, 1310b, 1310c, 1310d, 1310e, 1310f, 1310g, 1311, 1312, 1313, 1314, 1315, 1311a, 1312a, 1313a, 1314a, 1315a, 1316, 1317, 1318, 1319, 1316a, 1317a 1318a, 1319a) are made of graphene and/or Molybdenum disulphide, and said at least one electrically isolating portion (121, 122, 123, 221, 321, 421, 422, 423, 424, 425, 551, 552, 651, 652, 721, 722, 723, 851, 852, 920, 921, 922, 923, 924, 925, 926, 927, 921 a, 922a, 923a, 924a, 925a, 925a, 926a, 927a, 1120, 1121, 1122, 1123, 1124, 1125, 1126, 1127, 1121a, 1122a, 1123a, 1124a, 1125a, 1126a, 1127a, 1320, 1321, 1322, 1323, 1324, 1325, 1326, 1321 a, 1322a, 1323a, 1324a, 1325a, 1326a) is made of boron nitride, hydrogenated graphene or graphene oxide.

14. A sensor for translocating analytes, comprising a functional membrane including at least one functional layer defining at least one hole (131, 231, 331, 332, 431, 531, 631, 731, 831, 931, 1031, 1131, 1231, 1331, 1431, 1593, 1693) traversing the whole thickness of the functional membrane, wherein said at least one hole (131, 231, 331, 332, 431, 531, 631, 731, 831, 931, 1031, 1131, 1231, 1331, 1431, 1593, 1693) has a cross-section at a nano-meter or micro-meter scale and is configured and arranged for allowing the pass there through of a target translocating analyte (500, 600, 800, 1000, 1200, 1400, 1520, 1620) contained in a fluid coming from a first fluidic channel or first reservoir (151, 251, 351, 551, 651, 851, 1051, 1251, 1451, 1591, 1691) and going to a second fluidic channel or second reservoir (152, 252, 352, 552, 652, 852, 1052, 1252, 1452, 1592, 1692), wherein said first (151, 251, 351, 551, 651, 851, 1051, 1251, 1451, 1591, 1691) and second (152, 252, 352, 552, 652, 852, 1052, 1252, 1452, 1592, 1692) fluidic channels or reservoirs are to be fluidically connected to the at least one hole through a cross-section larger than the cross-section of the at least one hole (131, 231, 331, 332, 431, 531, 631, 731, 831, 931, 1031, 1131, 1231, 1331, 1431, 1593, 1693) at the interface with the functional membrane, **characterised in that** said sensor comprises a slow-down and manipulation mechanism including a control unit and an electrical generator with outputs connected to electrodes configured and arranged to generate and provide, under the control of the control unit, electrical signals to the electrodes which are suitable to generate a time-controlled non-uniform electric field in the at least one hole (131, 231, 331, 332, 431, 531, 631, 731, 831, 931, 1031, 1131, 1231, 1331, 1431, 1593, 1693) of the at least one functional layer, and/or in the vicinity thereof, to selectively produce dielectrophoretic forces which counter transversal forces initially governing the motion of the target translocating analyte (500, 600, 800, 1000, 1200, 1400, 1520, 1620) through the at least one hole (131, 231, 331, 332, 431, 531, 631, 731, 831, 931, 1031, 1131, 1231, 1331, 1431, 1593, 1693), to slow down the velocity, alter the motion pattern, retain/stop or re-collocate the target translocating analyte (500, 600, 800, 1000, 1200, 1400, 1520, 1620) in a controlled manner.

15. A method for sensing translocating analytes, comprising:

- performing measurements of ionic current blockade and/or of transverse and/or longitudinal electron current or local impedance changes of at least one plane of the inner volume of at least one hole (131, 231, 331, 332, 431, 531, 631, 731, 831, 931, 1031, 1131, 1231, 1331, 1431, 1593, 1693) defined in a functional layer of a functional membrane and traversing the whole thickness of said functional membrane, wherein said at least one hole (131, 231, 331, 332, 431, 531, 631, 731, 831, 931, 1031, 1131, 1231, 1331, 1431, 1593, 1693) has a cross-section at a sub-nanometer or nano-meter scale or with a value of up to 1mm,

wherein said measurements are performed while and result from the translocation or immobilization of a target translocating analyte (500, 600, 800, 1000, 1200, 1400, 1520, 1620) through the at least one hole (131, 231, 331, 332, 431, 531, 631, 731, 831, 931, 1031, 1131, 1231, 1331, 1431, 1593, 1693) coming from a first fluidic channel or first reservoir (651, 851, 1051, 1251, 1451, 1591, 1691) and going to a second fluidic channel or second reservoir (652, 852, 1052, 1252, 1452, 1592, 1692), wherein said first and second fluidic channels or reservoirs have a cross-section larger than the cross-section of the at least one hole (131, 231, 331, 332, 431, 531, 631, 731, 831, 931, 1031, 1131, 1231, 1331, 1431, 1593, 1693) at the interface with the functional membrane, **characterised in that** the method further comprises slowing down the velocity, altering the motion pattern, retaining/stopping or re-collocating the target translocating analyte (500, 600, 800, 1000, 1200, 1400, 1520, 1620) in a controlled manner, while passing through the at least one hole (131, 231, 331, 332, 431, 531, 631, 731, 831, 931, 1031, 1131, 1231, 1331, 1431, 1593, 1693) by generating a time-controlled non-uniform electric field in the at least one hole (131, 231, 331, 332, 431, 531, 631, 731, 831, 931, 1031, 1131, 1231, 1331, 1431, 1593, 1693) of the at least one functional layer, and/or in the vicinity thereof, to selectively produce dielectrophoretic forces which counter transversal forces initially governing the motion of the target translocating analyte (500, 600, 800, 1000, 1200, 1400, 1520, 1620) through the at least one hole (131, 231, 331, 332, 431, 531, 631, 731, 831, 931, 1031, 1131, 1231, 1331, 1431, 1593, 1693).

**Fig. 1**

**Fig. 2**

Fig. 3

EP 3 349 005 A1

A  411  421  412  D E F

415  422  416

425

B  421  421  D E F

423  415  416  424

425  431  425

C  D E F

**Fig. 4**

E  415  422  416

423  424

B  431  B

A  A

D  441  442

413  421  414

B  431  B

411  412

441  442

A  A

F  425

B  431  B

A  A

Fig. 5

Fig. 6

Fig. 7

Fig. 8

Fig. 9

Fig. 9

Fig. 10

Fig. 11

Fig. 11

Fig. 12

EP 3 349 005 A1

EP 3 349 005 A1

Fig. 13

Fig. 13

Fig. 14

Fig. 15

Fig. 16

EP 3 349 005 A1

Europäisches
Patentamt

European
Patent Office

Office européen
des brevets

# EUROPEAN SEARCH REPORT

Application Number

EP 17 38 2014

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | US 2014/360876 A1 (AKSIMENTIEV ALEKSEI [US]) 11 December 2014 (2014-12-11)<br>* the whole document * | 1-10<br><br>11<br>14,15 | INV.<br>G01N33/487 |
| X<br><br><br><br><br><br><br><br><br><br>Y<br>A | JIWOOK SHIM ET AL: "Graphene nanopores for nucleic acid analysis",<br>2012 12TH IEEE INTERNATIONAL CONFERENCE ON NANOTECHNOLOGY (IEEE-NANO 2012) : BIRMINGHAM, UNITED KINGDOM, 20 - 23 AUGUST 2012, IEEE, PISCATAWAY, NJ,<br>20 August 2012 (2012-08-20), pages 1-2, XP032260393,<br>DOI: 10.1109/NANO.2012.6322237<br>ISBN: 978-1-4673-2198-3<br>* the whole document * | 1-10,12, 13<br><br><br><br><br><br><br><br><br><br>11<br>14,15 | |
| X<br><br><br><br><br><br><br><br><br><br><br>Y<br>A | CHIA-JUNG CHANG ET AL: "Control single dsDNA molecule stretching and transportation by using virtual nanopore trapper",<br>NANO/MICRO ENGINEERED AND MOLECULAR SYSTEMS (NEMS), 2011 IEEE INTERNATIONAL CONFERENCE ON, IEEE,<br>20 February 2011 (2011-02-20), pages 772-775, XP031966022,<br>DOI: 10.1109/NEMS.2011.6017468<br>ISBN: 978-1-61284-775-7<br>* the whole document * | 14,15<br><br><br><br><br><br><br><br><br><br><br>11<br>1-10,12, 13 | **TECHNICAL FIELDS SEARCHED (IPC)**<br><br>G01N |

The present search report has been drawn up for all claims

| Place of search | Date of completion of the search | Examiner |
|---|---|---|
| The Hague | 13 June 2017 | Zwerger, Markus |

EPO FORM 1503 03.82 (P04C01)

## ANNEX TO THE EUROPEAN SEARCH REPORT
## ON EUROPEAN PATENT APPLICATION NO.

EP 17 38 2014

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2017

| Patent document cited in search report | Publication date | Patent family member(s) | Publication date |
|---|---|---|---|
| US 2014360876 A1 | 11-12-2014 | NONE | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2656508 A
- US 20130309776 A1
- US 20130037410 A1
- US 20150028846 A1

**Non-patent literature cited in the description**

- *Genomics Proteomics Bioinformatics,* 2015, vol. 13, 4
- *Small,* 2009, vol. 5, 2638
- *Nano Letters,* 2010, vol. 10, 2915
- *Nature,* 2010, vol. 467, 190
- *ACS Nano,* 2012, vol. 6 (10), 8583
- *Nano Letters,* 2012, vol. 12, 4869
- *Nature,* 2012, vol. 488, 627
- *Nature,* 2013, vol. 8, 119
- *Nano Letters,* 2013, vol. 13, 2668